# EUROPEAN PATENT APPLICATION

(11) **EP 2 805 948 A1**
(43) Date of publication of application: **26.11.2014**
(21) Application number: 13738250.3
(22) Date of filing: 21.01.2013
(51) Int. Cl.: C07D 405/12, C07D 215/14, C07D 491/052, A61K 31/4709, A61P 29/00, A61P 37/00, A61P 43/00, C07B 61/00

(54) **INDUSTRIAL PROCESS FOR PREPARATION OF 1,2-DIHYDROQUINOLINE DERIVATIVE OR A SALT THEREOF, AND INTERMEDIATE FOR PREPARATION THEREOF**

(30) Priority: 20.01.2012 JP 2012010106
(71) Applicant: Santen Pharmaceutical Co., Ltd, Osaka-shi, Osaka 533-8651 (JP); Kyowa Hakko Bio Co., Ltd., Tokyo 100-8185 (JP)
(72) Inventor: YAMAMOTO Noriyoshi, Ikoma-shi Nara 630-0101 (JP); OHNO Atsushi, Ikoma-shi Nara 630-0101 (JP); KUDOU Kazuhiro, Ikoma-shi Nara 630-0101 (JP); BAN Masakazu, Ikoma-shi Nara 630-0101 (JP); MIMURA Takashi, Tokyo 1008185 (JP); OHTANI Takashi, Sakai-shi Osaka 5998124 (JP)
(74) Representative: Bawden, Peter Charles
(86) International application number: PCT/JP2013/051103
(87) International publication number: WO 2013/108921

(57) **Abstract**

Provided is a method for producing a compound represented by formula (7) or a salt thereof: 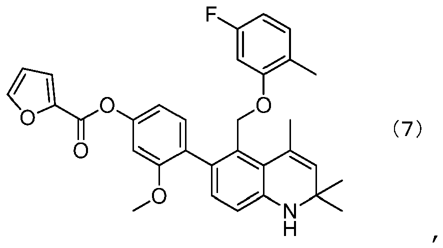 the method comprising the steps of:
removing a R¹ group and a R² group from a compound represented by formula (5) or a salt thereof: 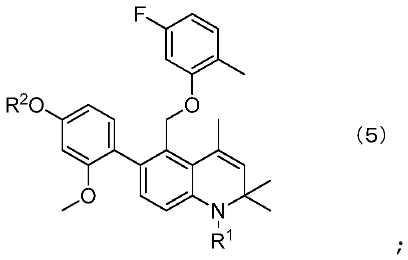 and
reacting the compound (in which R1 and R2 in formula (5) are hydrogen atoms) obtained in the removing step or a salt thereof with a compound represented by formula (d): 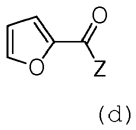 or a salt thereof in the presence of a base. The method is a method for producing a 1,2-dihydroquinoline derivative having glucocorticoid receptor binding activity or a salt thereof. This method does not require a purification step by column chromatography and is suitable for industrial production.

## Description

### Technical Field

The present invention relates to a method for industrially producing a 1,2-dihydroquinoline derivative having glucocorticoid receptor binding activity or a salt thereof, as well as intermediates for producing the same.

### Background Art

The compound represented by formula (7): 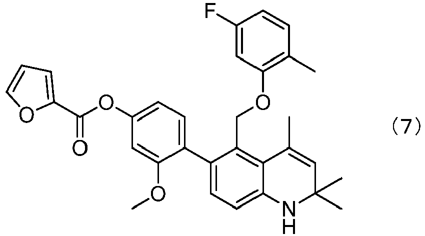 and salts thereof are known to have binding activity to the glucocorticoid receptor, and are reported to be produced by the following production flow (Patent Literature 1). LiAlH₄ represents lithium aluminum hydride, and MeI represents methyl iodide.

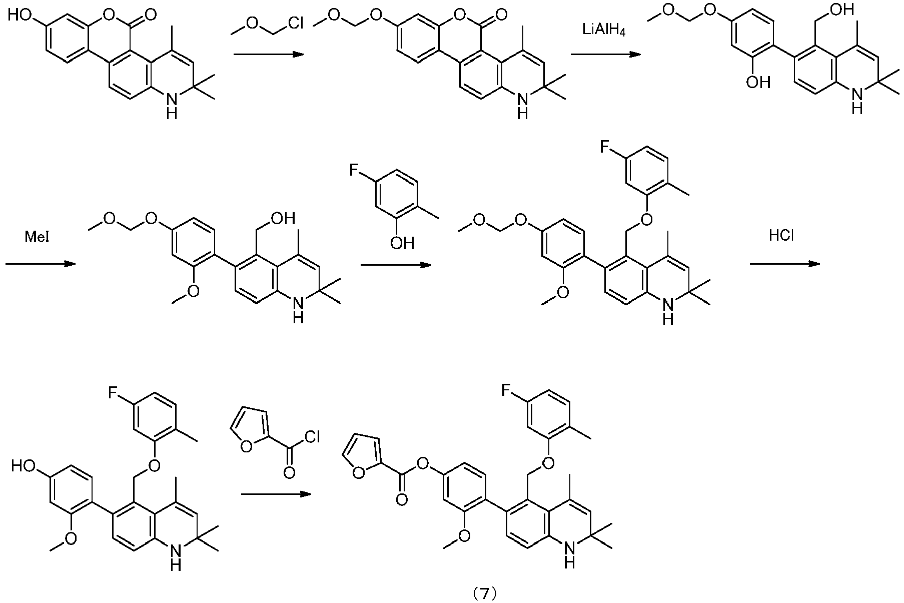

The production method shown in Patent Literature 1 requires a purification step by column chromatography in each production step, and achieves a moderate yield in each step. Hence, the production method has problems in terms of industrial production.

In addition, the compound represented by formula (7) or salts thereof are known to be useful as a prophylactic or therapeutic agent for inflammatory diseases and immune diseases (Patent Literature 2).

### Citation List

### Patent Literatures

Patent Literature 1: WO2008/059865A
Patent Literature 2: WO2009/139361A

### Summary of Invention

An object of the present invention is to provide a method for producing a 1,2-dihydroquinoline derivative having glucocorticoid receptor binding activity or a salt thereof, the method requiring no purification step by column chromatography and being suitable for industrial production, as well as to provide intermediates for the production thereof.

The present inventors have intensively studied a method for industrially producing a compound represented by formula (7) or a salt thereof. As a result, the present inventors have found a method (hereinafter, referred to as a "method of the present invention") for industrially producing a compound represented by formula (7) or a salt thereof at a good yield, in which the hydroxy group on the phenyl group in the 6-position of a 1,2-dihydroquinoline derivative or a salt thereof is protected with a specific protective group such as a p-methoxybenzyl group, a benzyl group, or a benzyloxymethyl group, so that the need for a column chromatography purification step of each production intermediate is eliminated, and each production intermediate can be isolated and purified by filtering and washing steps. In addition, the present inventors have found a production method in which, by using the protective group, the hydroxy group on the 6-phenyl group is protected and deprotected at high yields. Moreover, the present inventors have found novel 1,2-dihydroquinoline derivatives and salts thereof (hereinafter, referred to as "compounds of the present invention") as production intermediates. These findings have led to the completion of the present invention.

Specifically, the present invention is as follows.
(i) A method for producing a compound represented by formula (7) or a salt thereof: 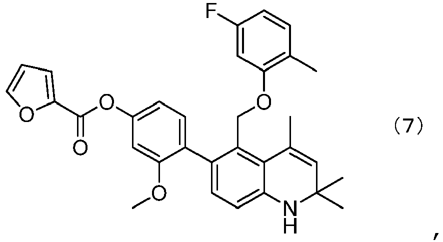 the method comprising the steps of:
   removing a R¹ group and a R² group from a compound represented by formula (5) or a salt thereof, provided that the R¹ group is removed only if the R¹ group is not a hydrogen atom: 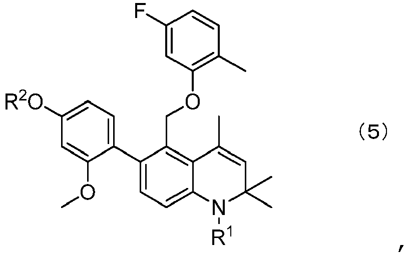 wherein R¹ represents a hydrogen atom or the same group as R², and R² represents an arylalkyl group, an arylalkyloxyalkyl group, or a substituted silyl group; and
   reacting a compound represented by formula (6) or a salt thereof obtained in the removal step: 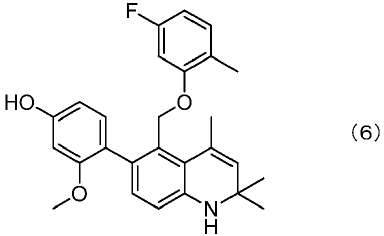 with a compound represented by formula (d) or a salt thereof: 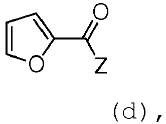 wherein Z represents a leaving group, in the presence of a base.
(ii) The production method according to (i), wherein
   the compound represented by formula (5) or the salt thereof is produced by reacting a compound represented by formula (4) or a salt thereof: 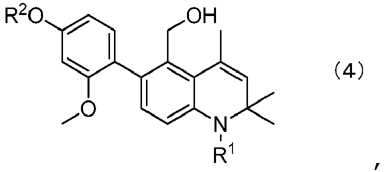 wherein R¹ and R² are as defined in formula (5),
   with a compound represented by formula (c) or a salt thereof: 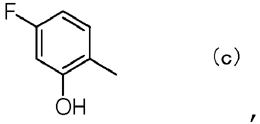 in the presence of a phosphine compound and a diazo compound.
(iii) The production method according to (ii), wherein
   the compound represented by formula (4) or the salt thereof is produced by reacting a compound represented by formula (3) or a salt thereof: 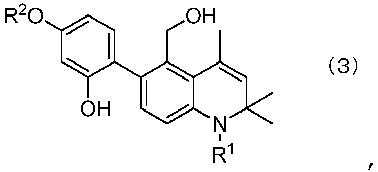 wherein R¹ and R² are as defined in formula (5),
   with a compound represented by formula (b) or a salt thereof:

   MeY (b),

   wherein Me represents a methyl group and Y represents a leaving group,
   in the presence of an acid or a base.
(iv) The production method according to (iii), wherein
   the compound represented by formula (3) or the salt thereof is produced by subjecting a compound represented by formula (2) or a salt thereof: 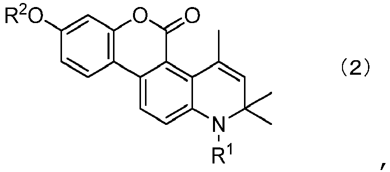 wherein R¹ and R² are as defined in formula (5),
   to a reduction treatment using a reducing agent.
(v) The production method according to (iv), wherein
   the compound represented by formula (2) or the salt thereof is produced by reacting a compound represented by formula (1) or a salt thereof: 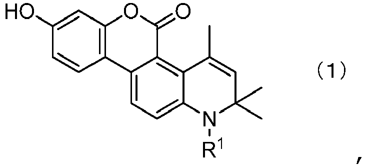 wherein R¹ represents a hydrogen atom,
   with a compound represented by formula (a) or a salt thereof:

   R²X (a),

   wherein R² is as defined in formula (5) and X represents a leaving group,
   in the presence of at least one selected from the group consisting of acids, bases, and halides.
(vi) The production method according to any one of (i) to (v), wherein
   R² is a p-methoxybenzyl group, a benzyl group, or a benzyloxymethyl group.
(vii) The production method according to (i), wherein
   the reaction for removing the R¹ group and the R² group is a reaction under an acidic condition or a catalytic reduction reaction in the presence of hydrogen.
(viii) The production method according to (ii), wherein
   the phosphine compound is triphenylphosphine or tributylphosphine, and the diazo compound is diethyl azodicarboxylate or diisopropyl azodicarboxylate.
(ix) The production method according to (iv), wherein
   the reducing agent is lithium aluminum hydride or sodium bis(2-methoxyethoxy)aluminum hydride.
(x) A compound represented by formula (2) or a salt thereof: 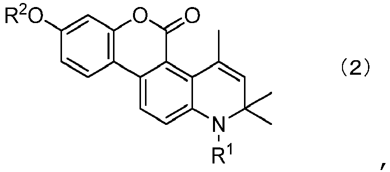 wherein R² represents an arylalkyl group, an arylalkyloxyalkyl group, or a substituted silyl group, and R¹ represents a hydrogen atom or the same group as R².
(xi) A compound represented by formula (3) or a salt thereof: 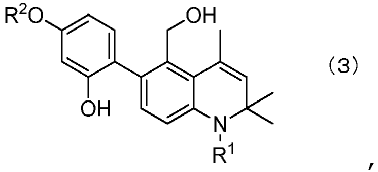 wherein R² represents an arylalkyl group, an arylalkyloxyalkyl group, or a substituted silyl group, and R¹ represents a hydrogen atom or the same group as R².
(xii) A compound represented by formula (4) or a salt thereof: 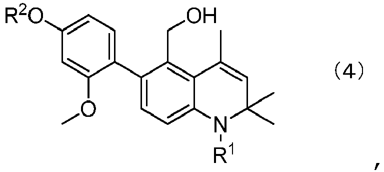 wherein R² represents an arylalkyl group, an arylalkyloxyalkyl group, or a substituted silyl group, and R¹ represents a hydrogen atom or the same group as R².
(xiii) A compound represented by formula (5) or a salt thereof: 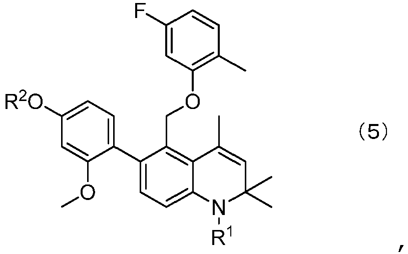 wherein R² represents an arylalkyl group, an arylalkyloxyalkyl group, or a substituted silyl group, and R¹ represents a hydrogen atom or the same group as R².
(xiv) The compound according to any one of (x) to (xiii) or a salt thereof, wherein
   R² is a p-methoxybenzyl group, a benzyl group, or a benzyloxymethyl group.
(xv) A compound or a salt thereof, the compound selected from the group consisting of
   8-(4-methoxybenzyloxy)-2,2,4-trimethyl-1,2-dihydro-6-oxa-1-azachrysen-5-one,
   6-[2-hydroxy-4-(4-methoxybenzyloxy)phenyl]-5-hydroxymethyl-1,2-dihydro-2,2,4-trimethylquinoline,
   5-hydroxymethyl-6-[2-methoxy-4-(4-methoxybenzyloxy)phenyl]-1,2-dihydro-2,2,4-trimethylquinoline,
   [5-[(5-fluoro-2-methylphenoxy)methyl]-6-[(2-methoxy-4-(4-me thoxybenzyloxy)phenyl)]-1,2-dihydro-2,2,4-trimethyl quinoline, and
   [5-[(5-fluoro-2-methylphenoxy)methyl]-6-(4-hydroxy-2-methoxyphenyl)-1,2-dihydro-2,2,4-trimethylquinoline, or a salt thereof.

The present invention makes it possible to provide a method for industrially producing a compound represented by formula (7) or a salt thereof at a good yield, in which the hydroxy group on the phenyl group in the 6-position of a 1,2-dihydroquinoline derivative or a salt thereof is protected with a specific protective group such as a p-methoxybenzyl group, a benzyl group, or a benzyloxymethyl group, so that the need for a column chromatography purification step of each production intermediate is eliminated, and each production intermediate is isolated and purified by filtering and washing steps. In addition, the present invention makes it possible to provide a production method in which, by using the protective group, the hydroxy group on the 6-phenyl group is protected and deprotected at high yields. Moreover, the present invention makes it possible to provide novel 1,2-dihydroquinoline derivatives and salts thereof as production intermediates.

### Description of Embodiments

Definitions of atoms, groups, rings, and the like used in this description are described in detail below. In addition, when a definition of a term below is also used to define another term, a preferred range and a particularly preferred range of the definition are also applied to the definition of the other term.

A "halogen atom" refers to a fluorine atom, a chlorine atom, a bromine atom, or an iodine atom.

A "lower alkyl group" refers to a linear or branched alkyl group having 1 to 8, preferably 1 to 6, and particularly preferably 1 to 4 carbon atoms. Specific examples thereof include a methyl group, an ethyl group, a n-propyl group, a n-butyl group, a n-pentyl group, a n-hexyl group, a n-heptyl group, a n-octyl group, an isopropyl group, an isobutyl group, a sec-butyl group, a tert-butyl group, a iso-pentyl group, and the like.

An "aryl group" refers to a residue obtainable by removing one hydrogen atom from a monocyclic aromatic hydrocarbon group or a bicyclic or tricyclic condensed polycyclic aromatic hydrocarbon having 6 to 14 carbon atoms. Specific examples thereof include a phenyl group, a naphthyl group, an anthryl group, a phenanthryl group, and the like.

An "arylalkyl group" refers to a group in which one or multiple hydrogen atoms of a lower alkyl group are substituted by one or multiple aryl groups. Specific examples thereof include a benzyl group, a bromobenzyl group, a chlorobenzyl group, a nitrobenzyl group, a dinitrobenzyl group, a dichlorobenzyl group, a difluorobenzyl group, a fluorousalkoxybenzyl group, a trimethylsilylmethylbenzyl group, a cyanobenzyl group, a p-phenylbenzyl group, a p-methoxybenzyl group, a m-methoxybenzyl group, a 3,4-dimethoxybenzyl group, a diphenylmethyl group, a p,p'-dinitrobenzhydryl group, a 5-dibenzosuberyl group, an α-naphthyldiphenylmethyl group, a p-methoxyphenyl diphenylmethyl group, a di(p-methoxyphenyl)phenylmethyl group, a tri(p-methoxyphenyl)methyl group, a 4-(4'-bromo phenacyloxypheny)diphenylmethyl group, a 4,4',4"-tris(4,5-dichlorophthalimidophenyl)methyl group, a 4,4',4"-tris(levulinoyloxyphenyl)methyl group, a 4,4',4"-tris(benzoyloxyphenyl)methyl group, a 3-(imidazol-1-ylmethyl)bis(4',4"-dimethoxyphenyl)methyl group, a 1,1-bis (4-methoxyphenyl)-1'-pyrenylmethyl group, a 9-anthrylmethyl group, a 9-(9-phenyl)xanthenyl group, a 9-phenylthioxanthenyl group, a 2,6-dimethylbenzyl group, a 2,4-dimethylbenzyl group, a 2,6-dichlorobenzyl group, a 4-(dimethylamino)carbonylbenzyl group, a 4-methylsulfinylbenzyl group, a pentadienylnitrobenzyl group, a 2-phenyl-2-propyl group, a p-acrylaminobenzyl group, a p-azidebenzyl group, a 4-azide-3-chlorobenzyl group, a trifluoromethylbenzyl group, a p-siletanylbenzyl group, a 4-acetoxybenzyl group, a 4-(2-trimethylsilyl) ethoxymethoxybenzyl group, a pyrenylmethyl group, a tris(4-tert-butylphenyl)methyl group, a phenethyl group, a phenylpropyl group, a phenylpentyl group, a triphenylmethyl group, and the like.

An "arylalkyloxy group" refers to a group in which the hydrogen atom of a hydroxy group is substituted by an arylalkyl group. Specific examples thereof include a benzyloxy group, a bromobenzyloxy group, a chlorobenzyloxy group, a nitrobenzyloxy group, a dinitrobenzyloxy group, a dichlorobenzyloxy group, a difluorobenzyloxy group a fluorousalkoxybenzyloxy group, a trimethylsilyl methylbenzyloxy group, a p-cyanobenzyloxy group, a p-phenylbenzyloxy group, a p-methoxybenzyloxy group, a m-methoxybenzyloxy group, a 3,4-dimethoxybenzyloxy group, a diphenylmethyloxy group, a p,p'-dinitrobenzhydryloxy group, a 5-dibenzosuberyloxy group, an α-naphthyldiphenylmethyloxy group, a p-methoxyphenyldiphenylmethyloxy group, a di(p-methoxyphenyl)phenylmethyloxy group, a tri(p-methoxy phenyl)methyloxy group, a 4-(4'-bromophenacyloxy phenyl)diphenylmethyloxy group, a 4,4',4"-tris(4,5-dichlorophthalimidophenyl)methyloxy group, a 4,4',4"-tris(levulinoyloxyphenyl)methyloxy group, a 4,4',4"-tris (benzoyloxyphenyl)methyloxy group, a 3-(imidazol-1-ylmethyl)bis(4',4"-dimethoxyphenyl)methyloxy group, a 1,1-bis(4-methoxyphenyl)-1'-pyrenylmethyloxy group, a 9-anthryloxy group, a 9-(9-phenyl)xanthenyloxy group, a 9-(9-phenyl-10-oxo)anthryloxy group, a 2,6-dimethylbenzyloxy group, a 2,6-dichlorobenzyloxy group, a 4-(dimethylamino carbonyl)benzyloxy group, a 4-methylsulfinylbenzyloxy group, a pentadienylnitrobenzyloxy group, a 2-phenyl-2-propyloxy group, a p-acrylaminobenzyloxy group, a p-azidebenzyloxy group, a 4-azide-3-chlorobenzyloxy group, a trifluoromethylbenzyloxy group, a p-siletanylbenzyloxy group, a 4-acetoxybenzyloxy group, a 4-(2-trimethylsilyl)ethoxymethoxybenzyloxy group, a pyrenylmethyloxy group, a tris(4-tert-butylphenyl)methyloxy group, a phenethyloxy group, a phenylpropyloxy group, a phenylpentyloxy group, a triphenylmethyloxy group, and the like.

An "arylalkyloxyalkyl group" refers to a group in which a hydrogen atom of a lower alkyl group is substituted by an arylalkyloxy group. Specific examples thereof include a benzyloxymethyl group, a bromobenzyloxymethyl group, a chlorobenzyloxymethyl group, a nitrobenzyloxy methyl group, a dinitrobenzyloxymethyl group, a 2-benzyloxyethyl group, a dichlorobenzyloxymethyl group, a difluorobenzyloxymethyl group, a fluorousalkoxybenzyloxymethyl group, a trimethylsilylmethylbenzyloxymethyl group, a p-cyanobenzyloxymethyl group, a p-phenylbenzyloxymethyl group, a p-methoxybenzyloxymethyl group, a m-methoxybenzyloxymethyl group, a 3,4-dimethoxybenzyloxymethyl group, a diphenylmethyloxymethyl group, a p,p'-dinitro benzhydryloxymethyl group, a 5-dibenzosuberyloxymethyl group, an α-naphthyldiphenylmethyloxymethyl group, a p-methoxy phenyldiphenylmethyloxymethyl group, a di(p-methoxy phenyl)phenylmethyloxymethyl group, a tri(p-methoxy phenyl)methyloxymethyl group, a 4-(4'-bromophenacyl oxyphenyl)diphenylmethyloxymethyl group, a 4,4',4"-tris (4,5-dichlorophthalimidophenyl)methyloxymethyl group, a 4,4',4"-tris(levulinoyloxyphenyl)methyloxymethyl group, a 4,4',4"-tris(benzoyloxyphenyl)methyloxymethyl group, a 3-(imidazol-1-ylmethyl)bis(4',4"-dimethoxyphenyl)methyloxy methyl group, a 1,1-bis(4-methoxyphenyl)-1'-pyrenylmethyloxymethyl group, a 9-anthryloxymethyl group, a 9-(9-phenyl)xanthenyloxymethyl group, a 9-(9-phenyl-10-oxo) anthryloxymethyl group, a 2, 6-dimethyl benzyloxymethyl group, a 2,6-dichlorobenzyloxymethyl group, a 4-(dimethylaminocarbonyl)benzyloxymethyl group, a 4-methyl sulfinylbenzyloxymethyl group, a pentadienyl nitrobenzyloxymethyl group, a 2-phenyl-2-propyloxymethyl group, a p-acrylaminobenzyloxymethyl group, a p-azidebenzyloxymethyl group, a 4-azide-3-chloro benzyloxymethyl group, a trifluoromethylbenzyloxymethyl group, a p-siletanylbenzyloxymethyl group, a phenethyloxymethyl group, a phenethyloxyethyl group, a phenylpropyloxymethyl group, a phenylpentyloxymethyl group, a (phenyldimethylsilyl)methoxymethyl group, a 2-methoxybenzyl oxymethyl group, and the like.

An "aryloxyalkyl group" refers to a group in which a hydrogen atom of a lower alkyl group is substituted by an aryloxyalkyl group. Specific examples thereof include a (4-methoxyphenoxy)methyl group and the like.

A "substituted silyl group" refers to a silyl group having any three substituents selected from lower alkyl groups and phenyl groups. Specific examples thereof include a trimethylsilyl group, a triethylsilyl group, a tert-butyldimethylsilyl group, a tert-butyldiphenylsilyl group, a dimethylisopropylsilyl group, a diethylisopropylsilyl group, a dimethylthexylsilyl group, a 2-norbornyldimethylsilyl group, a tribenzylsilyl group, a triphenylsilyl group, a diphenylsilylmethyl group, a di-tert-butylmethylsilyl group, a bis(tert-butyl) -1-pyrenylmethoxysilyl group, a tris(trimethylsilyl)silyl group, a (2-hydroxystyryl)dimethylsilyl group, a (2-hydroxystyryl)diisopropylsilyl group, a tert-butylmethoxyphenylsilyl group, a tert-butoxydiphenylsilyl group, a 1,1,3,3-tetraisopropyl-3-[2-(triphenylmethoxy)ethoxy] disiloxan-1-yl group, a fluorous silyl group, a triisopropylsilyl group, and the like.

A "leaving group" refers to a substituent to be removed by a reaction. Specific examples thereof include halogen atoms such as a fluorine atom, a chlorine atom, a bromine atom, and an iodine atom; lower alkyl sulfonyloxy groups such as a methanesulfonyloxy group, a chloromethylsulfonyloxy group, and a trifluoromethanesulfonyloxy group; aryl sulfonyloxy groups such as a benzenesulfonyloxy group and a p-toluenesulfonyloxy group; a cyano group; a nitro group, trichloroacetimidate, and the like.

The compounds represented by formulae (1), (2), (3), (4), (5), (6), and (7) and raw materials and reagents used in the method of the present invention each may be in the form of a "salt" with an acid or a base. Specific examples thereof include salts with inorganic acids such as hydrochloric acid, hydrobromic acid, hydroiodic acid, nitric acid, sulfuric acid, and phosphoric acid; salts with organic acids such as carbonic acid, acetic acid, fumaric acid, maleic acid, succinic acid, citric acid, tartaric acid, adipic acid, gluconic acid, glucoheptonic acid, glucuronic acid, terephthalic acid, methanesulfonic acid, lactic acid, hippuric acid, 1,2-ethanedisulfonic acid, isethionic acid, lactobionic acid, oleic acid, pamoic acid, polygalacturonic acid, stearic acid, tannic acid, trifluoromethanesulfonic acid, benzenesulfonic acid, p-toluenesulfonic acid, 10-camphorsulfonic acid, sulfuric acid monolauryl ester, sulfuric acid monomethyl ester, naphthalenesulfonic acid, and sulfosalicylic acid; quaternary ammonium salts with methyl bromide, methyl iodide, and the like; salts with halogen ions such as a bromine ion, a chlorine ion, and an iodine ion; salts with alkali metals such as lithium, sodium, and potassium; salts with alkaline earth metals such as calcium and magnesium; salts with metals such as copper, iron, and zinc; salts with ammonia; salts with organic amines such as triethylenediamine, 2-aminoethanol, 2,2-iminobis(ethanol), 1-deoxy-1-(methylamino)-2-D-sorbitol, 2-amino-2-(hydroxymethyl)-1,3-propanediol, procaine, and N,N-bis(phenylmethyl)-1,2-ethane diamine; and the like.

The compounds represented by formulae (1), (2), (3), (4), (5), (6), and (7) and the raw materials and reagents used in the method of the present invention each may be in the form of a hydrate or a solvate.

When any of the compounds represented by formulae (1), (2), (3), (4), (5), (6), and (7) and the raw materials and reagents used in the method of the present invention has geometrical isomers or optical isomers, these isomers are also within the scope of the present invention.

When any of the compounds represented by formulae (1), (2), (3), (4), (5), (6), and (7) and the raw materials and reagents used in the method of the present invention undergoes proton tautomerization, the tautomer is also included in the present invention.

The compounds represented by formulae (1), (2), (3), (4), (5), (6), and (7), the raw materials and reagents used in the method of the present invention, and hydrates and solvates thereof may be in the form of crystals. When polymorphs and a polymorphic group (polymorphic system) of the crystals exists, these polymorphs and the polymorphic group (polymorphic system) are also included in the present invention. Here, the polymorphic group (polymorphic system) means crystal forms in all stages in a case where the crystal form changes depending on the conditions and state during production, crystallization, storage, or the like of the crystals (note that this state also includes a state of being formulated into a pharmaceutical preparation), as well as the entire process.

The production method of the present invention is described below.

### <Step 1>

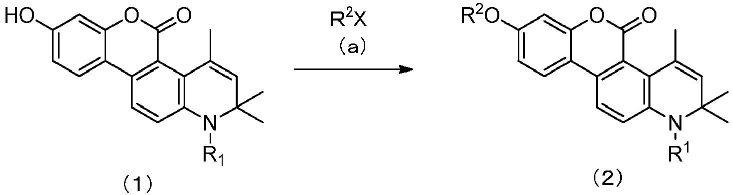

Step 1 is a step of reacting a compound represented by formula (1) or a salt thereof with a compound represented by formula (a) : R²X, or a salt thereof in the presence of at least one selected from the group consisting of acids, bases, and halides, to produce a compound represented by formula (2) or a salt thereof. Note that the compound represented by formula (1) or a salt thereof can be obtained by a method described in International Publication No. WO 2008/059865.

In formulae (a) and (2), R² may be an arylalkyl group such as a benzyl group, a bromobenzyl group, a chlorobenzyl group, a nitrobenzyl group, a dinitrobenzyl group, a dichlorobenzyl group, a difluorobenzyl group, a fluorousalkoxybenzyl group, a trimethylsilylmethylbenzyl group, a cyanobenzyl group, a cyanobenzyl group, a p-phenylbenzyl group, a p-methoxybenzyl group, a m-methoxybenzyl group, a 3,4-dimethoxybenzyl group, a diphenylmethyl group, a p,p'-dinitrobenzhydryl group, a 5-dibenzosuberyl group, an α-naphthyldiphenylmethyl group, a p-methoxyphenyldiphenylmethyl group, a di(p-methoxyphenyl) phenylmethyl group, a tri(p-methoxyphenyl)methyl group, a 4-(4'-bromophenacyloxyphenyl)diphenylmethyl group, a 4,4',4"-tris(4,5-dichlorophthalimidophenyl)methyl group, a 4,4',4"-tris(levulinoyloxyphenyl)methyl group, a 4,4',4"-tris(benzoyloxyphenyl)methyl group, a 3-(imidazol -1-ylmethyl)bis(4',4"-dimethoxyphenyl)methyl group, a 1,1-bis(4-methoxyphenyl)-1'-pyrenylmethyl group, a 9-anthrylmethyl group, a 9-(9-phenyl)xanthenyl group, a 9-(9-phenyl-10-oxo) anthryl group, a 2,6-dimethylbenzyl group, a 2,6-dichlorobenzyl group, a 4-(dimethylamino)carbonylbenzyl group, a 4-methylsulfinylbenzyl group, a pentadienyl nitrobenzyl group, a 2-phenyl-2-propyl group, a p-acrylaminobenzyl group, a p-azidebenzyl group, a 4-azide-3-chlorobenzyl group, a trifluoromethylbenzyl group, a p-siletanylbenzyl group, a 4-acetoxybenzyl group, a 4-(2-trimethylsilyl)ethoxymethoxybenzyl group, a pyrenylmethyl group, a tris(4-tert-butylphenyl)methyl group, a phenethyl group, a phenylpropyl group, a phenylpentyl group, or a triphenylmethyl group; an arylalkyloxyalkyl group such as a benzyloxymethyl group, a bromobenzyloxymethyl group, a chlorobenzyloxymethyl group, a nitrobenzyloxy group methyl, a dinitrobenzyloxymethyl group, a 2-benzyloxyethyl group, a dichlorobenzyloxymethyl group, a difluorobenzyloxymethyl group, a fluorousalkoxybenzyloxymethyl group, a trimethylsilylmethylbenzyloxymethyl group, a p-cyano benzyloxymethyl group, a p-phenylbenzyloxymethyl group, a p-methoxybenzyloxymethyl group, a m-methoxybenzyloxymethyl group, a 3,4-dimethoxybenzyloxymethyl group, a diphenylmethyloxymethyl group, a p,p'-dinitro benzhydryloxymethyl group, a 5-dibenzosuberyloxymethyl group, an α-naphthyldiphenylmethyloxymethyl group, a p-methoxy phenyldiphenylmethyloxymethyl group, a di(p-methoxy phenyl)phenylmethyloxymethyl group, a tri(p-methoxyphenyl)methyloxymethyl group, a 4-(4'-bromo phenacyloxyphenyl)diphenylmethyloxymethyl group, a 4,4',4"-tris(4,5-dichlorophthalimidophenyl)methyloxymethyl group, a 4,4',4"-tris(levulinoyloxyphenyl)methyloxymethyl group, a 4,4',4"-tris(benzoyloxyphenyl)methyloxymethyl group, a 3-(imidazol-1-ylmethyl)bis(4',4"-dimethoxyphenyl)methyloxymethyl group, a 1,1-bis(4-methoxyphenyl)-1'-pyrenylmethyloxymethyl group, a 9-anthryloxymethyl group, a 9-(9-phenyl)xanthenyloxymethyl group, a 9-(9-phenyl-10-oxo)anthryloxymethyl group, a 2,6-dimethylbenzyloxymethyl group, a 2,6-dichloro benzyloxymethyl group, a 4-(dimethylamino carbonyl)benzyloxymethyl group, a 4-methylsulfinyl benzyloxymethyl group, a pentadienylnitrobenzyloxymethyl group, a 2-phenyl-2-propyloxymethyl group, a p-acrylaminobenzyloxymethyl group, a p-azidebenzyloxymethyl group, a 4-azide-3-chlorobenzyloxymethyl group, a trifluoromethylbenzyloxymethyl group, a p-siletanyl benzyloxymethyl group, a phenethyloxymethyl group, a phenethyloxyethyl group, a phenylpropyloxymethyl group, a phenylpentyloxymethyl group, a (phenyldimethylsilyl) methoxymethyl group, or a 2-methoxybenzyloxymethyl group; an aryloxyalkyl group such as a (4-methoxyphenoxy)methyl group; or a substituted silyl group such as a trimethylsilyl group, a triethylsilyl group, a tert-butyldimethylsilyl group, a tert-butyldiphenylsilyl group, a dimethylisopropylsilyl group, a diethylisopropylsilyl group, a dimethylthexylsilyl group, a 2-norbornyldimethylsilyl group, a tribenzylsilyl group, a triphenylsilyl group, a diphenylsilylmethyl group, a di-tert-butylmethylsilyl group, a bis(tert-butyl)-1-pyrenylmethoxysilyl group, a tris(trimethylsilyl)silyl group, a (2-hydroxystyryl)dimethylsilyl group, a (2-hydroxystyryl) diisopropylsilyl group, a tert-butylmethoxyphenylsilyl group, a tert-butoxydiphenylsilyl group, a 1,1,3,3-tetraisopropyl-3-[2-(triphenylmethoxy)ethoxy]disiloxan-1-yl group, a fluorous silyl group, or a triisopropylsilyl group. R² is preferably a benzyloxy group, a p-methoxybenzyloxy group, or a benzyloxymethyl group, and more preferably a p-methoxybenzyloxy group or a benzyloxymethyl group.

In formula (1), R¹ represents a hydrogen atom.

In formula (2), R¹ represents a hydrogen atom or R².

In formula (a), X represents a leaving group, and may be any substituent, as long as the substituent can be removed by the reaction. Examples thereof include halogen atoms such as a fluorine atom, a chlorine atom, a bromine atom, and an iodine atom; lower alkyl sulfonyloxy groups such as a methanesulfonyloxy group, a chloromethylsulfoxyoxy group, and a trifluoromethanesulfonyloxy group; aryl sulfonyloxy groups such as a benzenesulfonyloxy group and a p-toluenesulfonyloxy group; a cyano group; a nitro group; trichloroacetimidate; carbonyldioxyphenyl; and the like. X is preferably a halogen atom, and further preferably a chlorine atom.

The compound represented by formula (a): R²X, or a salt thereof is used in an amount of 1 equivalent or more, preferably 1 to 2 equivalents, and further preferably 1.2 to 1.5 equivalents, relative to the compound represented by formula (1) or the salt thereof.

Examples of the acid used in this step include inorganic acids such as hydrochloric acid, sulfuric acid, nitric acid, phosphoric acid, hydrogen bromide, and hydrofluoric acid; organic acids such as trifluoroacetic acid, methanesulfonic acid, trifluoromethanesulfonic acid, benzenesulfonic acid, p-toluenesulfonic acid, and aminosulfonic acid; Lewis acids such as boron tribromide, boron trichloride, boron trifluoride, and aluminum chloride; and the like.

The acid is used in this step in an amount of 1 equivalent or more, preferably 1 to 5 equivalents, and further preferably 2 to 3 equivalents, relative to the compound represented by formula (1) or the salt thereof.

Examples of the base used in this step include inorganic bases including alkali metal carbonates such as sodium carbonate, potassium carbonate, cesium carbonate, and lithium carbonate; alkali metal hydrogen carbonates such as sodium hydrogen carbonate, potassium hydrogen carbonate, and lithium hydrogen carbonate; alkali metal hydroxides such as sodium hydroxide, potassium hydroxide, barium hydroxide, and lithium hydroxide; alkali metal hydrides such as lithium hydride, sodium hydride, and potassium hydride; alkali metal fluorides such as sodium fluoride and potassium fluoride; and the like; as well as alkali metal alkoxides such as sodium methoxide, sodium ethoxide, potassium methoxide, potassium ethoxide, potassium t-butoxide, and lithium methoxide; and organic bases such as N-methylmorpholine, triethylamine, tripropylamine, tributylamine, diisopropylethylamine, dicyclohexylamine, N-methylpiperidine, pyridine, 4-pyrrolidinopyridine, picoline, 4-(N,N-dimethylamino)pyridine, 2,6-di(t-butyl) -4-methylpyridine, quinoline, N,N-dimethylaniline, N,N-diethylaniline, 1,5-diazabicyclo[4.3.0]non-5-ene (DBN), 1,4-diazabicyclo[2.2.2]octane (DABCO), 1,8-diazabicyclo [5.4.0]undec-7-ene (DBU), and N,N,N',N',N",N"-hexamethylphosphoric triamide (HMPA). The base is preferably an alkali metal carbonate, and further preferably potassium carbonate.

The base is used in this step in an amount of 1 equivalent or more, preferably 1 to 5 equivalents, and further preferably 2 to 3 equivalents, relative to the compound represented by formula (1) or the salt thereof.

Examples of the halide used in this step include iodides such as potassium iodide, sodium iodide, lithium iodide, antimony triiodide, nitrogen triiodide, arsenic triiodide, triiodides, boron triiodide potassium bromide, silicon tetraiodide, potassium tetraiodomercurate(II), zinc iodide, aluminum iodide, ammonium iodide, yttrium(III) iodide, cadmium iodide, gallium(III) iodide, calcium iodide, silver (I) iodide, chromium iodide, germanium diiodide, cobalt(II) iodide, samarium(II) iodide, zirconium(IV) iodide, mercury iodide, mercury(II) iodide, mercury(I) iodide, hydrogen iodide, tin iodide, tin(II) iodide, strontium iodide, cesium iodide, tantalum(V) iodide, iron(II) iodide, copper(I) iodide, thorium(IV) iodide, lead(II) iodide, niobium(V) iodide, nickel(II) iodide, vanadium(III) iodide, barium iodide, beryllium iodide, magnesium iodide, manganese(II) iodide, and rubidium iodide; bromides such as potassium bromide, sodium bromide, lithium bromide, antimony tribromide, arsenic tribromide, boron tribromide, iodine tribromide, zinc bromide, aluminum bromide, ammonium bromide, cadmium bromide, calcium bromide, gold(III) bromide, silver(I) bromide, cobalt(II) bromide, mercury(II) bromide, strontium bromide, cesium bromide, iron(III) bromide, tetraethylammonium bromide, copper(I) bromide, lead(II) bromide, nickel(II) bromide, platinum(II) bromide, platinum(IV) bromide, vanadium(III) bromide, barium bromide, beryllium bromide, magnesium bromide, radium bromide, and rubidium bromide. The halide is preferably an iodide, and further preferably potassium iodide, sodium iodide, or lithium iodide.

The halide is used in this step in an amount of 0.1 equivalents or more, preferably 1 to 5 equivalents, and further preferably 1 to 3 equivalents, relative to the compound represented by formula (1) or the salt thereof.

The solvent used in this step is not particularly limited, as long as the solvent does not inhibit the reaction, and is capable of dissolving the starting substance to some degree. Examples of the solvent include aromatic hydrocarbons such as benzene, toluene, and xylene; halogenated hydrocarbons such as dichloromethane, chloroform, carbon tetrachloride, dichloroethane, chlorobenzene, and dichlorobenzene; ethers such as diethyl ether, diisopropyl ether, tetrahydrofuran, cyclopentyl methyl ether, methyl tert-butyl ether, dioxane, dimethoxyethane, and diethylene glycol dimethyl ether; lower-alkylcarboxylic acid esters such as ethyl acetate and isopropyl acetate; amides such as formamide, N,N-dimethylformamide, N,N-dimethylacetamide, N-methyl-2-pyrrolidinone, and hexamethylphosphorotriamide; sulfoxides such as dimethyl sulfoxide and sulfolane; lower alcohols such as methanol, ethanol, propanol, isopropanol, butanol, isobutanol, and tert-butanol; water; and mixture solvents of any ones of these solvents. The solvent is preferably a mixture of an amide and water, and further preferably a mixture of N,N-dimethylformamide and water.

Although the reaction temperature varies depending on the raw material compound and the reaction reagent, the reaction is conducted at 0°C to 100°C, and preferably 0°C to 50°C.

Although the reaction time varies depending on the reaction temperature, the raw material compound, the reaction reagent, and the type of the solvent used, the reaction time is generally 1 minute to 48 hours, and preferably 5 minutes to 5 hours.

The target compound can be obtained as follows. Specifically, after completion of the reaction, the reaction mixture is cooled, and a solvent such as water, ammonia water, or methanol is added to the reaction mixture with stirring under ice-cooling. Then, the precipitated solid is filtered. Alternatively, after completion of the reaction, the reaction mixture is subjected to extraction and concentration. Then, to the obtained residue, a solvent, such as toluene or ethyl acetate, from which the target compound can be recrystallized is added, and the precipitated solid is filtered and dried. Note that, after the precipitated solid is filtered, the precipitated solid is preferably washed with toluene, ethyl acetate, water, or the like.

If necessary, the obtained target compound can be purified by a usual method, for example, recrystallization, reprecipitation, or the like.

### <Step 2>

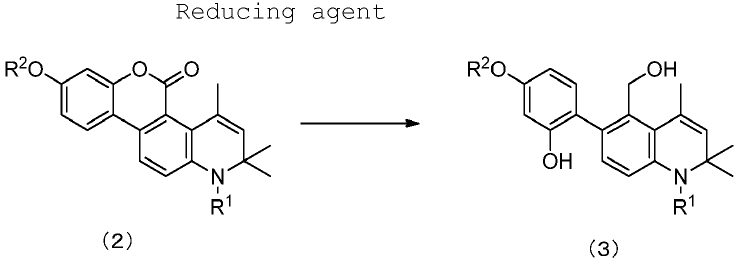

Step 2 is a step of subjecting the compound represented by formula (2) or a salt thereof to a reduction treatment using a reducing agent, to produce a compound represented by formula (3) or a salt thereof.

In formulae (2) and (3), R¹ and R² have the same definitions as those of R¹ and R² described in <Step 1> above.

Examples of the reducing agent used in this step include alkali metal borohydrides such as sodium borohydride and lithium borohydride; aluminum hydride compounds such as lithium aluminum hydride and lithium triethoxyaluminum hydride; hydride reagents such as sodium hydrogen telluride; and the like. The reducing agent is preferably an aluminum hydride compound, and more preferably lithium aluminum hydride or sodium bis(2-methoxyethoxy)aluminum hydride. Industrially, the reducing agent is further preferably sodium bis(2-methoxyethoxy)aluminum hydride.

The reducing agent is used in an amount of 1 equivalent or more and preferably 1 to 10 equivalents relative to the compound represented by formula (2) or the salt thereof.

The solvent used in this step is not particularly limited, as long as the solvent does not inhibit the reaction, and is capable of dissolving the starting substance to some degree. Examples of the solvent include aromatic hydrocarbons such as benzene, toluene, and xylene; halogenated hydrocarbons such as dichloromethane, chloroform, carbon tetrachloride, dichloroethane, chlorobenzene, and dichlorobenzene; ethers such as diethyl ether, diisopropyl ether, tetrahydrofuran, cyclopentyl methyl ether, methyl tert-butyl ether, dioxane, dimethoxyethane, and diethylene glycol dimethyl ether; lower-alkylcarboxylic acid esters such as ethyl acetate and isopropyl acetate; amides such as formamide, N,N-dimethylformamide, N,N-dimethylacetamide, N-methyl-2-pyrrolidinone, and hexamethylphosphorotriamide; sulfoxides such as dimethyl sulfoxide and sulfolane; lower alcohols such as methanol, ethanol, propanol, isopropanol, butanol, isobutanol, and tert-butanol; water; and mixture solvents of any ones of these solvents. The solvent is preferably tetrahydrofuran.

Although the reaction temperature varies depending on the raw material compound and the reaction reagent, the reaction is conducted at 0°C to 100°C, and preferably 0°C to 50°C.

Although the reaction time varies depending on the reaction temperature, the raw material compound, the reaction reagent, and the type of the solvent used, the reaction time is generally 1 minute to 48 hours, and preferably 5 minutes to 5 hours.

The target compound can be obtained as follows. Specifically, after completion of the reaction, the reaction mixture is cooled, and a solvent such as water or an aqueous potassium sodium tartrate solution is added to the reaction mixture with stirring under ice-cooling. Then, the precipitated solid is filtered. Alternatively, after completion of the reaction, the reaction mixture is subjected to extraction and concentration. Then, to the obtained residue, a solvent, such as 2-butanol, ethyl acetate, toluene, from which the target compound can be recrystallized is added, and the precipitated solid is filtered and dried. Note that after the precipitated solid is filtered, the solid is preferably washed with toluene, ethyl acetate, 2-butanol, or the like.

If necessary, the obtained target compound can be purified by a usual method, for example, recrystallization, reprecipitation, or the like.

### <Step 3>

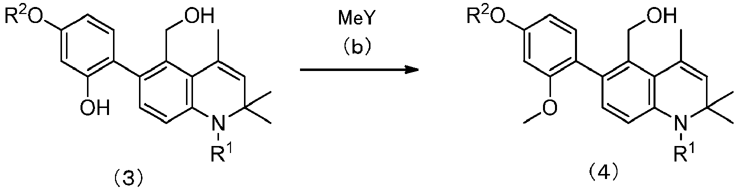

Step 3 is a step of reacting the compound represented by formula (3) or a salt thereof with a compound represented by formula (b) : MeY, or a salt thereof in the presence of an acid or a base, to produce a compound represented by formula (4) or a salt thereof.

In formulae (3) and (4), R¹ and R² have the same definitions as those of R¹ and R² described in <Step 1> above.

In formula (b), Me represents a methyl group, and Y represents a leaving group. The leaving group may be any substituent, as long as the substituent can be removed by the reaction. Examples of the leaving group include halogen atoms such as a fluorine atom, a chlorine atom, a bromine atom, and an iodine atom; lower alkyl sulfonyloxy groups such as a methanesulfonyloxy group, a chloromethylsulfoxyoxy group, and a trifluoromethanesulfonyloxy group; aryl sulfonyloxy groups such as a benzenesulfonyloxy group and a p-toluenesulfonyloxy group; a cyano group; a nitro group; and the like. The leaving group is preferably a halogen atom, and further preferably an iodine atom.

The compound represented by formula (b) : MeY, or a salt thereof is used in this step in an amount of 1 equivalent or more and preferably 1 to 2 equivalents relative to the compound represented by formula (3) or the salt thereof.

Examples of the acid used in this step include inorganic acids such as hydrochloric acid, sulfuric acid, nitric acid, phosphoric acid, hydrogen bromide, and hydrofluoric acid; organic acids such as trifluoroacetic acid, methanesulfonic acid, trifluoromethanesulfonic acid, benzenesulfonic acid, p-toluenesulfonic acid, and aminosulfonic acid; Lewis acids such as boron tribromide, boron trichloride, boron trifluoride, and aluminum chloride; and the like.

The acid is used in this step in an amount of 1 equivalent or more, preferably 1 to 5 equivalents, and further preferably 2 to 3 equivalents, relative to the compound represented by formula (3) or the salt thereof.

Example of the base used in this step include inorganic bases including alkali metal carbonates such as sodium carbonate, potassium carbonate, cesium carbonate, and lithium carbonate; alkali metal hydrogen carbonates such as sodium hydrogen carbonate, potassium hydrogen carbonate, and lithium hydrogen carbonate; alkali metal hydroxides such as sodium hydroxide, potassium hydroxide, barium hydroxide, and lithium hydroxide; alkali metal hydrides such as lithium hydride, sodium hydride, and potassium hydride; alkali metal fluorides such as sodium fluoride and potassium fluoride; and the like; as well as alkali metal alkoxides such as sodium methoxide, sodium ethoxide, potassium methoxide, potassium ethoxide, potassium t-butoxide, and lithium methoxide; and organic bases such as N-methylmorpholine, triethylamine, tripropylamine, tributylamine, diisopropylethylamine, dicyclohexylamine, N-methylpiperidine, pyridine, 4-pyrrolidinopyridine, picoline, 4-(N,N-dimethylamino)pyridine, 2,6-di(t-butyl)-4-methylpyridine, quinoline, N,N-dimethylaniline, N,N-diethylaniline, 1,5-diazabicyclo[4.3.0]non-5-ene (DBN), 1,4-diazabicyclo[2.2.2]octane (DABCO), and 1,8-diazabicyclo [5.4.0]undec-7-ene (DBU). The base is preferably an alkali metal carbonate, and further preferably potassium carbonate.

The base is used in this step in an amount of 1 equivalent or more, preferably 1 to 5 equivalents, and further preferably 1 to 3 equivalents, relative to the compound represented by formula (3) or the salt thereof.

The solvent used is not particularly limited, as long as the solvent does not inhibit the reaction, and is capable of dissolving the starting substance to some degree. Examples of the solvent include aromatic hydrocarbons such as benzene, toluene, and xylene; halogenated hydrocarbons such as dichloromethane, chloroform, carbon tetrachloride, dichloroethane, chlorobenzene, and dichlorobenzene; ethers such as diethyl ether, diisopropyl ether, tetrahydrofuran, cyclopentyl methyl ether, methyl tert-butyl ether, dioxane, dimethoxyethane, and diethylene glycol dimethyl ether; lower-alkylcarboxylic acid esters such as ethyl acetate and isopropyl acetate; amides such as formamide, N,N-dimethylformamide, N,N-dimethylacetamide, N-methyl-2-pyrrolidinone, and hexamethylphosphorotriamide; sulfoxides such as dimethyl sulfoxide and sulfolane; lower alcohols such as methanol, ethanol, propanol, isopropanol, butanol, isobutanol, and tert-butanol; water; and mixture solvents of any ones of these solvents. The solvent is preferably N,N-dimethylformamide, and further preferably a mixture solvent of N,N-dimethylformamide and water.

Although the reaction temperature varies depending on the raw material compound and the reaction reagent, the reaction is conducted at -78°C to 100°C, and preferably -10°C to 50°C.

Although the reaction time varies depending on the reaction temperature, the raw material compound, the reaction reagent, and the type of the solvent used, the reaction time is generally 1 minute to 48 hours, and preferably 5 minutes to 5 hours.

The target compound can be obtained as follows. Specifically, after completion of the reaction, the reaction mixture is cooled, and a solvent such as water, ammonia water, or methanol is added to the reaction mixture with stirring under ice-cooling. Then, the precipitated solid is filtered. Alternatively, after completion of the reaction, the reaction mixture is subjected to extraction and concentration. Then, to the obtained residue, a solvent, such as n-heptane or ethyl acetate, from which the target compound can be recrystallized is added, and the precipitated solid is filtered and dried. Note that after the precipitated solid is filtered, the solid is preferably washed with ethyl acetate, n-heptane, or the like.

If necessary, the obtained target compound can be purified by a usual method, for example, recrystallization, reprecipitation, or the like.

### <Step 4>

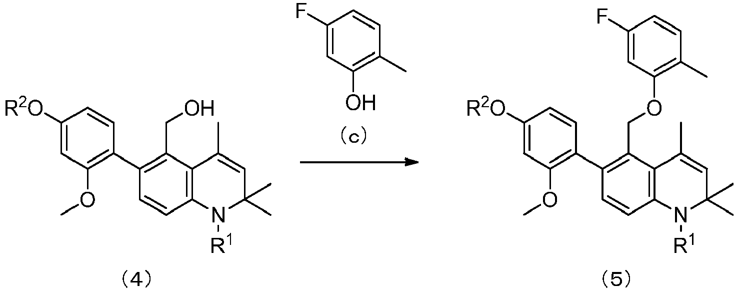

Step 4 is a step of reacting the compound represented by formula (4) or a salt thereof with a compound represented by formula (c) or a salt thereof in the presence of a phosphine compound and a diazo compound, to produce a compound represented by formula (5) or a salt thereof.

In formulae (4) and (5), R¹ and R² have the same definitions as those of R¹ and R² described in <Step 1> above.

In this step, the compound represented by formula (c) or the salt thereof is used in an amount of 1 equivalent or more, preferably 1 to 5 equivalents, and further preferably 1 to 2 equivalents per equivalent of the compound represented by formula (4) or the salt thereof.

Examples of the phosphine compound used in this step include triphenylphosphine, tributylphosphine, dicyclohexylphenylphosphine, diethylphenylphosphine, 4-(dimethylamino)phenyldiphenylphosphine, 4-diphenyl phosphinomethylpolystyrene resin, diphenyl-2-pyridyl phosphine, isopropyldiphenylphosphine, phenoxydiphenyl phosphine, tri-tert-butylphosphine, tricyclohexylphosphine, trihexylphosphine, tri-n-octylphosphine, and the like. The phosphine compound is preferably triphenylphosphine or tributylphosphine, and further preferably tributylphosphine.

The phosphine compound is used in an amount of 1 equivalent or more, and preferably 1 to 5 equivalents, relative to the compound represented by formula (4) or the salt thereof.

Examples of the diazo compound used in this step include 1,1'-azobis(N,N-dimethylformamide), 1,1'-(azodicarbonyl)dipiperidine, bis(2,2,2-trichloroethyl) azodicarboxylate, di-tert-butyl azodicarboxylate, dibenzyl azodicarboxylate, diethyl azodicarboxylate, diisopropyl azodicarboxylate, dimethyl azodicarboxylate, and the like. The diazo compound is preferably diethyl azodicarboxylate or diisopropyl azodicarboxylate, and further preferably diisopropyl azodicarboxylate.

The diazo compound is used in an amount of 1 equivalent or more and preferably 1 to 5 equivalents, relative to the compound represented by formula (4) or the salt thereof.

Note that a reagent such as (cyanomethylene) tributylphosphorane can be used instead of the phosphine compound and the diazo compound.

The solvent used is not particularly limited, as long as the solvent does not inhibit the reaction, and is capable of dissolving the starting substance to some degree. Examples of the solvent include aromatic hydrocarbons such as benzene, toluene, and xylene; halogenated hydrocarbons such as dichloromethane, chloroform, carbon tetrachloride, dichloroethane, chlorobenzene, and dichlorobenzene; ethers such as diethyl ether, diisopropyl ether, tetrahydrofuran, cyclopentyl methyl ether, methyl tert-butyl ether, dioxane, dimethoxyethane, and diethylene glycol dimethyl ether; lower-alkylcarboxylic acid esters such as ethyl acetate and isopropyl acetate; amides such as formamide, N,N-dimethylformamide, N,N-dimethylacetamide, N-methyl-2-pyrrolidinone, and hexamethylphosphorotriamide; sulfoxides such as dimethyl sulfoxide and sulfolane; lower alcohols such as methanol, ethanol, propanol, isopropanol, butanol, isobutanol, and tert-butanol; water; and mixture solvents of any ones of these solvents. The solvent is preferably tetrahydrofuran.

Although the reaction temperature varies depending on the raw material compound and the reaction reagent, the reaction temperature is within a range from -10°C to the boiling point of the solvent used, and is preferably 0°C to 40°.

Although the reaction time varies depending on the reaction temperature, the raw material compound, the reaction reagent, and the type of the solvent used, the reaction time is generally 1 minute to 48 hours, and preferably 5 minutes to 5 hours.

The target compound can be obtained as follows. Specifically, after completion of the reaction, the reaction mixture is cooled, and a solvent such as isopropyl alcohol is added to the reaction mixture with stirring under ice-cooling. Then, the precipitated solid is filtered. Alternatively, after completion of the reaction, the reaction mixture is subjected to extraction and concentration. Then, to the obtained residue, a solvent, such as isopropyl alcohol or ethyl acetate, from which the target compound can be recrystallized is added. Then, the precipitated solid is filtered and dried. Note that after the precipitated solid is filtered, the solid is preferably washed with ethyl acetate, isopropyl alcohol, or the like.

If necessary, the obtained target compound can be purified by a usual method, for example, recrystallization, reprecipitation, or the like.

### <Step 5>

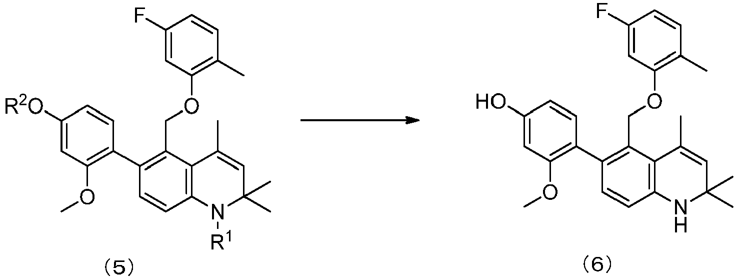

Step 5 is a step of subjecting the compound represented by formula (5) or a salt thereof to a reaction for removing the R¹ group and the R² group (provided that the R¹ group is removed only if the R¹ group is not a hydrogen atom), to produce a compound represented by formula (6) or a salt thereof.

In formula (5), R¹ and R² have the same definitions as those of R¹ and R² described in <Step 1> above.

The reaction for removing the R¹ group and the R² group (provided that the R¹ group is removed only if the R¹ group is not a hydrogen atom) is a so-called deprotection reaction, and can be conducted by a usual method (see Protective Groups in Organic Synthesis Fourth Edition, 2007). Examples of the reaction include a reaction under an acidic condition using an inorganic acid such as hydrochloric acid, sulfuric acid, nitric acid, phosphoric acid, hydrogen bromide, hydrofluoric acid, or hydrogen chloride; an organic acid such as trifluoroacetic acid, methanesulfonic acid, trifluoromethanesulfonic acid, benzenesulfonic acid, p-toluenesulfonic acid, or aminosulfonic acid; or a Lewis acid such as boron tribromide, boron trichloride, boron trifluoride, or aluminum chloride; a reaction using 2,3-dichloro-5,6-dicyano-p-benzoquinone, ammonium cerium(IV) nitrate, or the like; a catalytic reduction reaction in the presence of hydrogen using a catalyst such as palladium carbon, platinum, or Raney nickel in an alcohol such as methanol or ethanol, an ether such as ether, tetrahydrofuran, or dioxane, a fatty acid such as acetic acid, or a mixture solvent of any ones of these organic solvents with water; and the like. The reaction is preferably a reaction under an acidic condition using hydrochloric acid. The acid is used in this step in an amount of 1 equivalent or more, preferably 1 to 20 equivalents, and further preferably 5 to 10 equivalents, relative to the compound represented by formula (5) or the salt thereof.

The solvent used is not particularly limited, as long as the solvent does not inhibit the reaction, and is capable of dissolving the starting substance to some degree. Examples of the solvent include aromatic hydrocarbons such as benzene, toluene, and xylene; halogenated hydrocarbons such as dichloromethane, chloroform, carbon tetrachloride, dichloroethane, chlorobenzene, and dichlorobenzene; ethers such as diethyl ether, diisopropyl ether, tetrahydrofuran, cyclopentyl methyl ether, methyl tert-butyl ether, dioxane, dimethoxyethane, and diethylene glycol dimethyl ether; lower-alkylcarboxylic acid esters such as ethyl acetate and isopropyl acetate; amides such as formamide, N,N-dimethylformamide, N,N-dimethylacetamide, N-methyl-2-pyrrolidinone, and hexamethylphosphorotriamide; sulfoxides such as dimethyl sulfoxide and sulfolane; lower alcohols such as methanol, ethanol, propanol, isopropanol, butanol, isobutanol, and tert-butanol; water; and mixture solvents of any ones of these solvents. The solvent is preferably ethyl acetate, and further preferably a mixture solvent of ethyl acetate and methanol.

Although the reaction temperature varies depending on the raw material compound and the reaction reagent, the reaction is conducted at -78°C to 100°C, and preferably -20°C to 70°C.

Although the reaction time varies depending on the reaction temperature, the raw material compound, the reaction reagent, and the type of the solvent used, the reaction time is generally 1 minute to 48 hours, and preferably 5 minutes to 5 hours.

The target compound can be obtained by filtering and drying a solid precipitated in the reaction mixture. Note that after the solid precipitated in the reaction mixture is filtered, the solid is preferably washed with an organic solvent such as ethyl acetate.

If necessary, the obtained target compound can be purified by a usual method, for example, recrystallization, reprecipitation, or the like.

### <Step 6>

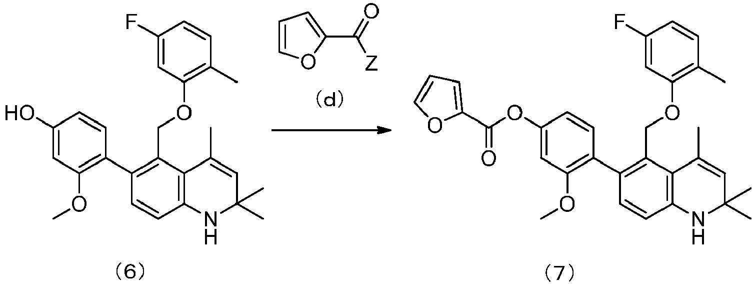

Step 6 is a step of reacting the compound represented by formula (6) or a salt thereof with a compound represented by formula (d), wherein Z represents a leaving group, or a salt thereof in the presence of a base, to produce a compound represented by formula (7) or a salt thereof.

In formula (d), Z represents a leaving group, and may be any substituent, as long as the substituent can be removed by the reaction. Examples of the leaving group include halogen atoms such as a fluorine atom, a chlorine atom, a bromine atom, and an iodine atom; lower alkyl sulfonyloxy groups such as a methanesulfonyloxy group, a chloromethylsulfoxyoxy group, and a trifluoromethanesulfonyloxy group; aryl sulfonyloxy groups such as a benzenesulfonyloxy group and a p-toluenesulfonyloxy group; a cyano group; a nitro group; and the like. The leaving group is preferably a halogen atom, and further preferably a chlorine atom.

In this step, the compound represented by formula (d) or a salt thereof is used in an amount of 1 equivalent or more, preferably 1 to 5 equivalents, and further preferably 1 to 2 equivalents, per equivalent of the compound represented by formula (6) or the salt thereof.

Examples of the base used in this step include inorganic bases including alkali metal carbonates such as sodium carbonate, potassium carbonate, cesium carbonate, and lithium carbonate; alkali metal hydrogen carbonates such as sodium hydrogen carbonate, potassium hydrogen carbonate, and lithium hydrogen carbonate; alkali metal hydroxides such as sodium hydroxide, potassium hydroxide, barium hydroxide, and lithium hydroxide; alkali metal hydrides such as lithium hydride, sodium hydride, and potassium hydride; alkali metal fluorides such as sodium fluoride and potassium fluoride; and the like; as well as alkali metal alkoxides such as sodium methoxide, sodium ethoxide, potassium methoxide, potassium ethoxide, potassium t-butoxide, and lithium methoxide; and organic bases such as N-methylmorpholine, triethylamine, tripropylamine, tributylamine, diisopropylethylamine, dicyclohexylamine, N-methylpiperidine, pyridine, 4-pyrrolidinopyridine, picoline, 4-(N,N-dimethylamino)pyridine, 2,6-di(t-butyl)-4-methylpyridine, quinoline, N,N-dimethylaniline, N,N-diethylaniline, 1,5-diazabicyclo[4.3.0]non-5-ene (DBN), 1,4-diazabicyclo[2.2.2]octane (DABCO), and 1,8-diazabicyclo[5.4.0]undec-7-ene (DBU). The base is preferably an organic base, and further preferably triethylamine.

The base is used in this step in an amount of 1 equivalent or more, preferably 1 to 10 equivalents, and further preferably 2 to 3 equivalents, relative to the compound represented by formula (6) or the salt thereof.

The solvent used is not particularly limited, as long as the solvent does not inhibit the reaction, and is capable of dissolving the starting substance to some degree. Examples of the solvent include aromatic hydrocarbons such as benzene, toluene, and xylene; halogenated hydrocarbons such as dichloromethane, chloroform, carbon tetrachloride, dichloroethane, chlorobenzene, and dichlorobenzene; ethers such as diethyl ether, diisopropyl ether, tetrahydrofuran, cyclopentyl methyl ether, methyl tert-butyl ether, dioxane, dimethoxyethane, and diethylene glycol dimethyl ether; lower-alkylcarboxylic acid esters such as methyl acetate, ethyl acetate, isopropyl acetate, and isobutyl acetate; amides such as formamide, N,N-dimethylformamide, N,N-dimethylacetamide, N-methyl-2-pyrrolidinone, and hexamethylphosphorotriamide; sulfoxides such as dimethyl sulfoxide and sulfolane; lower alcohols such as methanol, ethanol, propanol, isopropanol, butanol, isobutanol, and tert-butanol; water; and mixture solvents of any ones of these solvents. The solvent is preferably an ether, and further preferably tetrahydrofuran.

Although the reaction temperature varies depending on the raw material compound and the reaction reagent, the reaction is conducted at -78°C to 100°C, and preferably at -20°C to 70°C.

Although the reaction time varies depending on the reaction temperature, the raw material compound, the reaction reagent, and the type of the solvent used, the reaction time is generally 1 minute to 48 hours, and preferably 5 minutes to 5 hours.

The target compound can be obtained as follows. Specifically, after completion of the reaction, the reaction mixture is cooled, and a solvent such as an aqueous sodium hydrogen carbonate solution is added to the reaction mixture with stirring under ice-cooling. Then, the precipitated solid is filtered. Alternatively, after completion of the reaction, the reaction mixture is subjected to extraction and concentration. Then, to the obtained residue, a solvent, such as a lower alcohol or a lower-alkylcarboxylic acid ester, from which the target compound can be recrystallized is added. Then, the precipitated solid is filtered and dried. Note that after the precipitated solid is filtered, the solid is preferably washed with a lower alcohol, a lower-alkylcarboxylic acid ester, or the like.

If necessary, the obtained target compound can be purified by a usual method, for example, recrystallization, reprecipitation, or the like.

Hereinafter, production examples of the present invention are described. Note that these examples are presented for better understanding of the present invention, and the scope of the present invention is not limited to these examples.

### [Example 1]

### <8-(4-Methoxybenzyloxy)-2,2,4-trimethyl-1,2-dihydro-6-oxa-1 -azachrysen-5-one (compound 2)>

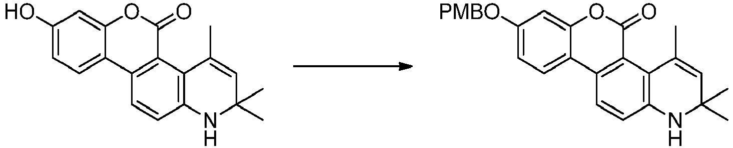

To a solution of 8-hydroxy-2,2,4-trimethyl-1,2-dihydro -6-oxa-1-azachrysen-5-one (Patent Literature 1, 1.00 g, 3.25 mmol) in N,N-dimethylformamide (12.0 mL), potassium carbonate (1.13 g, 8.13 mmol) and further 4-methoxybenzyl chloride (0.58 ml, 4.23 mmol) were added, followed by stirring with heating at an external temperature of 40°C for 5 hours. After being cooled, the reaction mixture was stirred under ice-cooling, and water (30.0 mL) was added. Then, the precipitated solid was filtered. The substance collected by filtration was further washed with water (8.00 mL), and then dried to obtain the title compound (1.39 g, yield: 87%).

| | |
|---|---|
| 8-(4-Methoxybenzylox y)-2,2,4-trimethyl-1 ,2-dihydro-6-oxa-1-a zachrysen-5-one (compound 2) | ¹H-NMR (500MHz, DMSO-D₆) δ: 1.21 (s, 6H), 1.95 (d, J=0.9 Hz, 3H), 3.76 (s, 3H), 5.10 (s, 2H), 5.42-5.43 (m, 1H), 6.83 (d, J=1.4 Hz, 1H), 6.96-6.97 (m, 4H), 7.15 (d, J=8.7 Hz, 1H), 7.40-7.42 (m, 2H), 7.90 (d, J=8.7 Hz, 1H), 8.01 (d, J=8.7 Hz, 1H). |
| 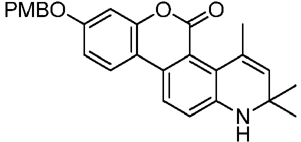 | |

### <6-[2-Hydroxy-4-(4-methoxybenzyloxy)phenyl]-5-hydroxymethyl -1,2-dihydro-2,2,4-trimethylquinoline (compound 3)>

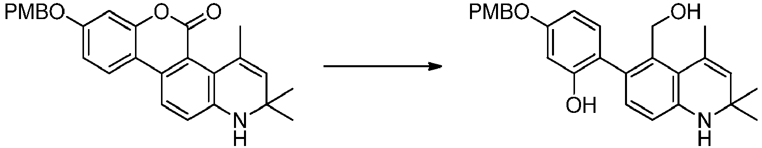

A mixture of 8-(4-methoxybenzyloxy)-2,2,4-trimethyl-1,2-dihydro-6-oxa-1-azachrysen-5-one (2.50 g, 5.85 mmol) and tetrahydrofuran (37.5 mL) was stirred at an ice-cold external temperature. When the internal temperature reached 5°C or below, a mixture solution of a sodium bis(2-methoxyethoxy)aluminum hydride-70% toluene solution (4.90 g, 17.7 mmol) and tetrahydrofuran (5.00 mL) was added, followed by stirring with heating at an at an external temperature of 40°C for 2 hours. After being cooled, the reaction mixture was stirred under ice-cooling, and a mixture solution of potassium sodium tartrate tetrahydrate (5.00 g, 17.7 mmol) and water (50.0 mL) was added thereto, followed by stirring for 30 minutes. Then, extraction was conducted by adding ethyl acetate (50 ml). The aqueous layer was washed with ethyl acetate (50 ml), and further the aqueous layer was washed with ethyl acetate (20 ml). Then, the organic layers were combined, and washed with water (25 ml) and with saturated aqueous sodium chloride (25 ml). The organic layer was concentrated, and ethyl acetate (7.50 ml) was added, followed by stirring at an external temperature of 0°C for 16 hours. Then, the precipitated solid was filtered. The substance collected by filtration was further washed with ethyl acetate (2.00 mL), and then dried to obtain the title compound (1.57 g, yield: 62%) .

| | |
|---|---|
| 6-[2-Hydroxy-4-(4-met hoxybenzyloxy)phenyl] -5-hydroxymethyl-1,2-dihydro-2,2,4-trimeth ylquinoline (compound 3) | ¹H-NMR (500MHz, DMSO-D₆) δ: 1.13 (s, 3H), 1.20 (s, 3H), 2.23 (d, J=1.4 Hz, 3H), 3.76 (s, 3H), 4.28 (d, J=11.0 Hz, 1H), 4.40 (s, 1H), 4.43 (d, J=11.0 Hz, 2H), 4.96 (s, 2H), 5.32 (m, 1H), 5.76 (d, J=1.8Hz, 1H), 6. 47 (dd, J=8.2, 2.3 Hz, 1H), 6.48 (s, 1H), 6.52 (d, J=8.2 Hz, 1H), 6.64 (d, J=8.2 Hz, 1H), 6.95-6.97 (m, 3H), 7.37-7.39 (m, 2H), 9.20 (s, 1H). |
| 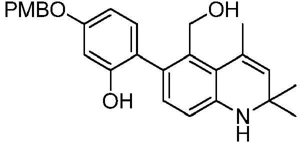 | |

### <5-Hydroxymethyl-6-[2-methoxy-4-(4-methoxybenzyloxy)phenyl] -1,2-dihydro-2,2,4-trimethylquinoline (compound 4)>

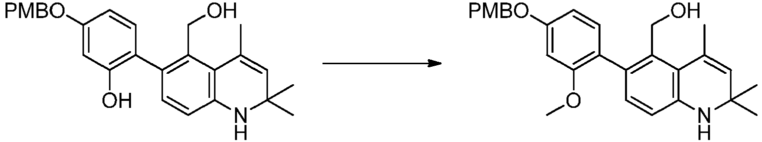

A solution obtained by mixing water (2.00 mL) and potassium carbonate (1.70 g, 12.3 mmol) with a solution of 6-[2-hydroxy-4-(4-methoxybenzyloxy)phenyl]-5-hydroxymethyl-1,2-dihydro-2,2,4-trimethylquinoline (2.66 g, 6.17 mmol) in N,N-dimethylformamide (40.0 mL) was cooled at an external temperature of 0°C, and methyl iodide (0.57 mL, 9.13 mmol) was added portionwise, followed by stirring for 24 hours. To the reaction mixture, ethyl acetate (32.0 mL), isopropyl ether (32.0 mL), and water (53.2 mL) were added, followed by phase separation. Extraction from the aqueous layer was conducted with ethyl acetate-isopropyl ether (1:1, 28.0 mL) twice. All the organic layers were combined, and washed with water (27.0 mL)×2 and with 25% aqueous sodium chloride (27.0 mL). The organic layer was concentrated to dryness, and then ethyl acetate (5.30 mL) and n-heptane (21.3 mL) were added, followed by stirring at an external temperature of 0°C for 1 hour. The precipitated solid was filtered. The substance collected by filtration was further washed with an ethyl acetate (2.00 mL)-n-heptane (2.00 mL) mixture solution, and then dried to obtain the title compound (2.45 g, yield: 89%).

| | |
|---|---|
| 5-Hydroxymethyl-6-[2 -methoxy-4-(4-methox ybenzyloxy)phenyl]-1 ,2-dihydro-2,2,4-tri methylquinoline (compound 4) | ¹H-NMR (400MHz, DMSO-D₆) δ: 1.13 (s, 3H), 1.20 (s, 3H), 2.23 (s, 3H), 3.65 (s, 3H), 3.77 (s, 3H), 4.15 (dd, J=12.2, 3.9 Hz, 1H), 4.30-4.34 (m, 1H), 4.42-4.48 (m, 1H), 5.03 (s, 2H), 5.32 (s, 1H), 5.78 (d, J=1.7 Hz, 1H), 6.48-6.53 (m, 1H), 6.58-6.66 (m, 3H), 6.94-6.99 (m, 2H), 7.04 (d, J=8.1 Hz, 1H), 7.02-7.06 (m, 2H). |
| 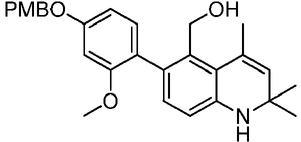 | |

### <[5-[(5-Fluoro-2-methylphenoxy)methyl]-6-[(2-methoxy-4-(4-m ethoxybenzyloxy)phenyl)]-1,2-dihydro-2,2,4-trimethylquinoli ne (compound 5)>

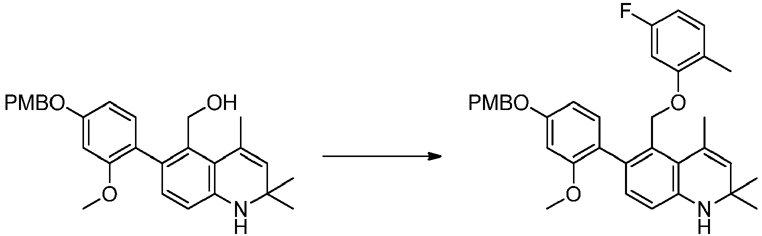

A solution obtained by mixing 5-fluoro-2-methylphenol (0.37 g, 2.96 mmol) and tributylphosphine (1.00 mL, 4.04 mmol) with a solution of 5-hydroxymethyl-6-[2-methoxy-4-(4-methoxybenzyloxy)phenyl]-1,2-dihydro-2,2,4-trimethylquinoline (1.20 g, 2. 69 mmol) in tetrahydrofuran (24.0 mL) was cooled at an external temperature of 0°C, and diisopropyl azodicarboxylate (0.79 mL, 4.04 mmol) was added thereto, followed by stirring at an external temperature of 20°C for 2 hours and 20 minutes. After the reaction mixture was concentrated, isopropyl alcohol (6.00 mL) was added thereto, followed by stirring at an external temperature of 0°C for 16 hours. Then, the precipitated solid was filtered. The substance collected by filtration was further washed with isopropyl alcohol (3.00 mL). The solid was taken out, and ethyl acetate (2.00 mL) was added thereto. The mixture was stirred at an external temperature of 50°C for 1 hour, and then at an external temperature of 0°C for 1 hour. The precipitated solid was filtered. The substance collected by filtration was further washed with ethyl acetate (2.00 mL), and then dried to obtain the title compound (0.80 g, yield: 53.1%).

| | |
|---|---|
| [5-[(5-Fluoro-2-methyl phenoxy)methyl]-6-[(2-methoxy-4-(4-methoxybe nzyloxy)phenyl)]-1,2-d ihydro-2,2,4-trimethyl quinoline (compound 5) | ¹H-NMR (400MHz, DMSO-D₆) δ: 1.02 (s, 3H), 1.14 (s,3H), 2.02 (s, 3H), 2.07 (d, J=1.2 Hz, 3H), 3.71 (s, 3H), 3.76 (s, 3H), 4.64 (d, J=12.2Hz, 1H), 5.02 (s, 2H), 5.09 (d, J=12.2Hz, 1H), 5.38 (s, 1H), 5.97 (d, J=1.5 Hz, 1H), 6.31 (dd, J=11.5, 2.4 Hz, 1H), 6.52 (td, J=8.3, 2.4 Hz, 1H), 6.58-6.64 (m, 2H), 6.68 (d, J=2.4 Hz, 1H), 6.74 (d, J=8.1 Hz, 1H), 6.93-6.98 (m, 2H), 6.99-7.05 (m, 1H), 7.07 (d, J=8.3 Hz, 1H), 7.37-7.42 (m, 2H). |
| 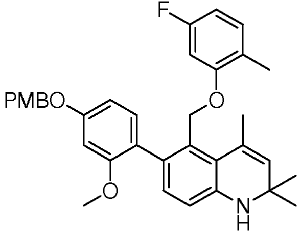 | |

### <[5-[(5-Fluoro-2-methylphenoxy)methyl]-6-(4-hydroxy-2-metho xyphenyl)-1,2-dihydro-2,2,4-trimethylquinoline hydrochloride (compound 6)>

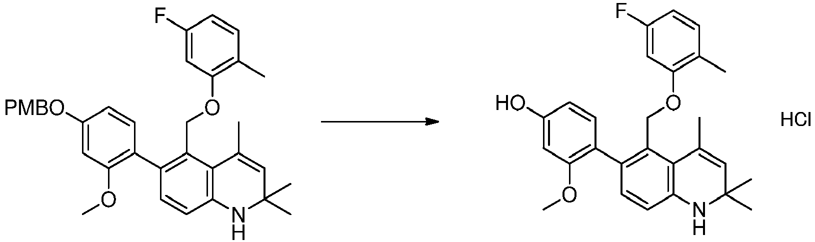

A solution of [5-[(5-fluoro-2-methylphenoxy) methyl]-6-[(2-methoxy-4-(4-methoxybenzyloxy)phenyl)]-1,2-di hydro-2,2,4-trimethylquinoline (0.65 g, 1.17 mmol) in ethyl acetate (5.50 mL) and methanol (1.30 mL) was cooled at an external temperature of 0°C, and a 4 N hydrogen chloride/ethyl acetate solution (1.50 mL, 5.85 mmol) was added thereto, followed by stirring at an external temperature of 50°C for 2 hours and 30 minutes. The temperature of the reaction mixture was returned to room temperature, and ethyl acetate (4.60 mL) was added to the reaction mixture, followed by stirring for 30 minutes. Then, the precipitated solid was filtered. The substance collected by filtration was further washed with ethyl acetate (4.00 mL), and then dried to obtain the title compound (0.49 g, yield: 88.4%).

| | |
|---|---|
| [5-[(5-Fluoro-2-methylp henoxy)methyl]-6-(4-hyd roxy-2-methoxyphenyl)-1 ,2-dihydro-2,2,4-trimet hylquinoline hydrochloride (compound 6) | ¹H-NMR (400MHz, DMSO-D₆) δ: 1.07 (s, 3H), 1.24 (s, 3H), 2.00 (s, 3H), 2.12 (s, 3H), 3.70 (s, 3H), 4.09 (brs, 2H), 4.70 (d, J=12.2 Hz, 1H), 5.13 (d, J=12.2 Hz, 1H), 5.58 (s, 1H), 6.28 (dd, J=11.3, 2.1 Hz, 1H), 6.41 (dd, J=8.1, 1.8 Hz, 1H), 6.48-6.56 (m, 2H), 6.89-7.08 (m, 4H), 9.64 (brs, 1H). |
| 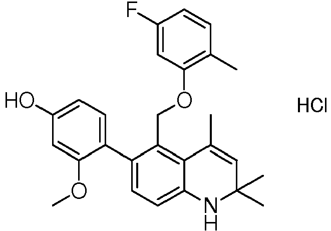 | |

### <5-(5-Fluoro-2-methylphenoxymethyl)-6-(4-hydroxy-2-methoxyp henyl)1,2-dihydro-2,2,4-trimethylquinoline hydrochloride (compound 6), another method>

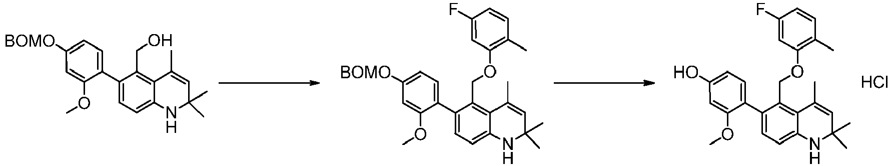

A solution obtained by mixing 5-fluoro-2-methylphenol (0.63 g, 4.99 mmol) and tributylphosphine (1.65 mL, 6.69 mmol) with a solution of 5-hydroxymethyl-6-(2-methoxy -4-benzyloxymethoxyphenyl)-1,2-dihydro-2,2,4-trimethylquino line (2.00 g, 4.49 mmol) in anhydrous tetrahydrofuran (45.0 mL) was cooled at an external temperature of 0°C, and diisopropyl azodicarboxylate (1.34 mL, 6.81 mmol) was added thereto, followed by stirring at room temperature for 2 hours and 14 minutes. To the reaction mixture, methanol (10.0 mL) was added, followed by concentration. Then, extraction was conducted by adding ethyl acetate (80.0 mL) and water (80.0 mL). The organic layer was washed with water (80.0 mL), and concentrated. A solution of the residue obtained by concentration in ethyl acetate (10.0 mL) and methanol (10.0 mL) was ice cooled, and a 4 N hydrogen chloride/ethyl acetate solution (5.60 mL, 22.4 mmol) was added thereto, followed by stirring at an external temperature of 40°C for 2 hours and 2 minutes. The temperature of the reaction mixture was returned to room temperature, and the reaction mixture was concentrated. Then, ethyl acetate (8.00 mL) was added thereto, followed by stirring at an external temperature of 4°C for 19 hours and 10 minutes. Then, the precipitated solid was filtered. The substance collected by filtration was further washed with ethyl acetate (6.00 mL), and then dried at 50°C under reduced pressure to obtain the title compound (1.21 g, yield: 57%).

| | |
|---|---|
| 5-(5-Fluoro-2-methylphenoxy methyl)-6-(2-methoxy-4-benz yloxymethoxyphenyl)-1,2-dih ydro-2,2,4-trimethylquinoli ne | ¹H-NMR (500MHz, DMSO-D₆) δ: 1.03 (s, 3H), 1.14 (s, 3H), 2.02 (s, 3H), 2.07 (d, J=1.2Hz, 3H), 3.70 (s, 3H), 4.65 (d, J=12.2Hz, 1H), 4.70 (s, 2H), 5.09 (d, J=12.2Hz, 1H), 5.35 (s, 2H), 5.38 (t, J=1.5 Hz, 1H), 5.96 (d, J=1.5 Hz, 1H), 6.32 (dd, J=11.5, 2.4 Hz, 1H), 6.52 (td, J=8.2, 2.4 Hz, 1H), 6.61 (d, J=8.2 Hz, 1H), 6.68 (dd, J=8.2, 2.4 Hz, 1H), 6.73 (d, J=2.4 Hz, 1H), 6.75 (d, J=7.9 Hz, 1H), 7.00-7.05 (m, 1H), 7.09 (d, J=8.2 Hz, 1H), 7.25-7.34 (m, 5H). |
| 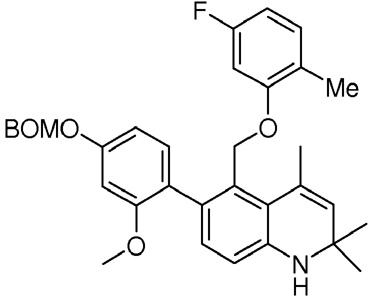 | |

| | |
|---|---|
| 5-(5-Fluoro-2-methylphenoxy methyl)-6-(4-hydroxy-2-meth oxyphenyl)-1,2-dihydro-2,2, 4-trimethylquinoline hydrochloride (compound 6) | ¹H-NMR (400MHz, DMSO-D₆) δ: 1.07 (s, 3H), 1.24 (s, 3H), 2.00 (s, 3H), 2.12 (s, 3H), 3.70 (s, 3H), 4.09 (brs, 2H), 4.70 (d, J=12.2 Hz, 1H), 5.13 (d, J=12.2 Hz, 1H), 5.58 (s, 1H), 6.28 (dd, J=11.3, 2.1 Hz, 1H), 6.41 (dd, J=8.1, 1.8 Hz, 1H), 6.48-6.56 (m, 2H), 6.89-7.08 (m, 4H), 9.64 (brs, 1H). |
| 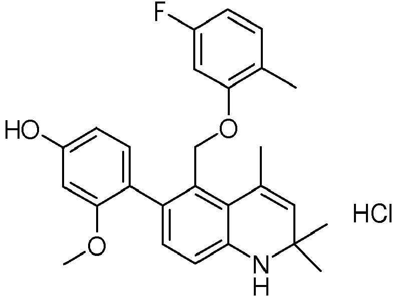 | |

### <4-[5-[(5-Fluoro-2-methylphenoxy)methyl]-1,2-dihydro-2,2,4-trimethyl-6-quinolinyl]-3-methoxyphenyl 2-furancarboxylate (compound 7)>

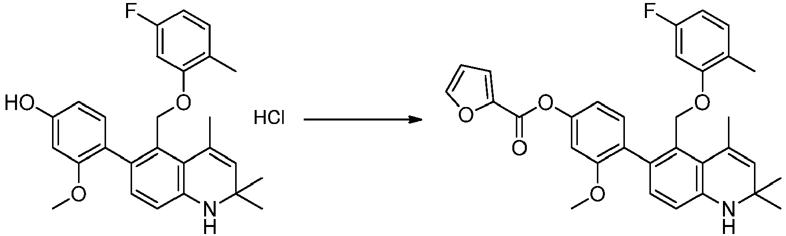

A solution of [5-[(5-fluoro-2-methylphenoxy) methyl]-6-(4-hydroxy-2-methoxyphenyl)-1,2-dihydro-2,2,4-tri methylquinoline hydrochloride (0.35 g, 0.75 mmol) in tetrahydrofuran (3.50 mL) was cooled at an external temperature of 0°C, and triethylamine (0.23 mL, 1.64 mmol) and 2-furoyl chloride (0.08 mL, 0.82 mmol) were added thereto, followed by stirring at room temperature for 2 hours and 30 minutes. The reaction mixture was cooled to 0°C, and ethyl acetate (3.50 mL) and an aqueous sodium hydrogen carbonate solution (sodium hydrogen carbonate: 0.29 g, water: 5.30 mL) were added thereto, followed by phase separation. The organic layer was washed with 5% aqueous sodium chloride (9.00 mL) five times. The organic layer was concentrated to dryness, and then ethyl acetate (0.37 mL) and isopropyl alcohol (1.10 mL) were added to the residue, and the residue was dissolved by stirring at an external temperature of 50°C for 15 minutes. Then, the temperature was returned to room temperature, followed by stirring for 16 hours. Then, the precipitated solid was filtered. The substance collected by filtration was further washed with isopropyl alcohol (1.40 mL), and then dried to obtain the title compound (0.25 g, yield: 63%).

| | |
|---|---|
| 4-[5-[(5-Fluoro-2-methylp henoxy)methyl]-1,2-dihydro-2,2,4-trimethyl-6-quino linyl]-3-methoxyphenyl 2-furancarboxylate (compound 7) | ¹H-NMR (400MHz, DMSO-D₆) δ: 1.06 (s, 3H), 1.15 (s, 3H), 2.02 (s, 3H), 2.07 (d, J=1.5 Hz, 3H), 3.73 (s, 3H), 4.63 (d, J=12.0 Hz, 1H), 5.09 (d, J=12.0Hz, 1H), 5.39-5.41 (m, 1H), 6.06 (d, J=1.5 Hz, 1H), 6.37 (dd, J=11.5, 2.4 Hz, 1H), 6.53 (td, J=8.3, 2.4 Hz, 1H), 6.64 (d, J=8.3,Hz, 1H), 6.81 (d, J=8.3 Hz, 1H), 6. 81 (dd, J=3. 7, 1.8 Hz, 1H), 6.87 (dd, J=8.3, 2.2 Hz, 1H), 7.02 (d, J=2.2 Hz, 1H), 7.04 (t, J=8.3 Hz, 1H), 7.23 (d, J=8.3 Hz, 1H), 7.57 (dd, J=3. 7, 0.73 Hz, 1H), 8.11 (dd, J=1.8, 0.73 Hz, 1H). |
| 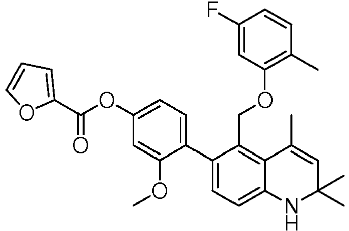 | |

### <8-Benzyloxymethoxy-2,2,4-trimethyl-1,2-dihydro-6-oxa-1-aza chrysen-5-one (compound 8)>

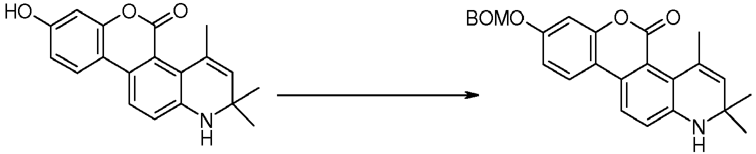

A mixture of 8-hydroxy-2,2,4-trimethyl-1,2-dihydro-6-oxa-1-azachrysen-5-one (Patent Literature 1, 2.50 g, 8.13 mmol) and anhydrous N,N-dimethylformamide (23.0 mL) was stirred under ice-cooling. When the internal temperature reached 0.2°C, 60% sodium hydride (0.32 g, 8.00 mmol) was added, followed by stirring for 30 minutes. To the reaction mixture, benzyl chloromethyl ether (1.25 mL, 9.10 mmol) was added, followed by stirring at room temperature for 1 hour and 42 minutes. Extraction from the reaction mixture was conducted by adding water (50.0 mL) and ethyl acetate (50.0 mL) under ice-cooling. After extraction from the aqueous layer with ethyl acetate (15.0 mL), the organic layers were combined, and washed with water (50.0 ml) four times. Then, the organic layer was concentrated. An operation in which methanol (7.5 mL) was added to the residue obtained by concentration and the mixture was concentrated was conducted twice. Then, methanol (7.5 mL) was added to the residue, followed by stirring under ice-cooling for 1 hour. The solid in the reaction mixture was filtered, washed with methanol (9.0 mL), and dried at 50°C under reduced pressure to obtain the title compound (2.91 g, yield: 84%).

| | |
|---|---|
| 8-Benzyloxymethoxy-2,2,4-trimethyl-1,2-dihydro-6-o xa-1-azachrysen-5-one (compound 8) | ¹H-NMR (500MHz, DMSO-D₆) δ: 1.22 (s, 6H), 1.95 (s, 3H), 4.72 (s, 2H), 5.40-5.44 (m, 3H), 6.85 (d, J=1.2 Hz, 1H), 7.00-7.04 (m, 2H), 7.16 (d, J=8.9 Hz, 1H), 7.27-7.37 (m, 5H), 7.92 (d, J=8.9 Hz, 1H), 8.05 (dd, J=8.1, 1.2 Hz, 1H). |
| 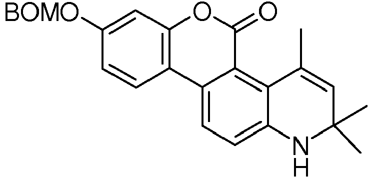 | |

### <5-Hydroxymethyl-6-(2-hydroxy-4-benzyloxymethoxyphenyl)-2,2 ,4-trimethyl-1,2-dihydroquinoline (compound 9)>

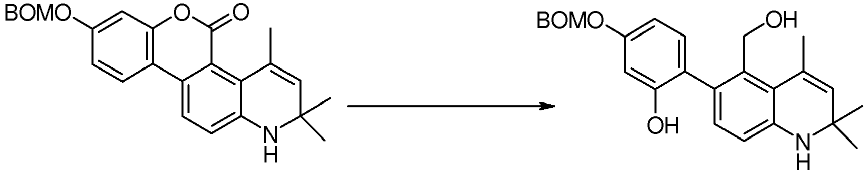

A mixture of 8-benzyloxymethoxy-2,2,4-trimethyl -1,2-dihydro-6-oxa-1-azachrysen-5-one (2.50 g, 5.85 mmol) and anhydrous tetrahydrofuran (30.0 mL) was stirred at an ice-cold external temperature. When the internal temperature reached 0.9°C or below, a mixture solution of a sodium bis(2-methoxyethoxy)aluminum hydride-70% toluene solution (5.01 g, 17.3 mmol) and anhydrous tetrahydrofuran (5.00 mL) was added, followed by stirring with heating at an external temperature of 40°C for 2 hours and 13 minutes. After being cooled, the reaction mixture was stirred under ice-cooling, and a mixture solution of potassium sodium tartrate tetrahydrate (5.51 g, 19.5 mmol) and water (50.0 mL) was added thereto, followed by stirring for 10 minutes. Then, extraction was conducted by adding ethyl acetate (50 ml). Extraction from the aqueous layer with ethyl acetate (7.50 ml) was conducted three times, and then the organic layers were combined, and washed with water (50.0 ml). The organic layer was concentrated, and an operation in which toluene (7.50 ml) was added and the mixture was concentrated was conducted three times. To the residue obtained by concentration, toluene (5.00 ml) was added, followed by stirring at an external temperature of 4°C for 14 hours and 40 minutes. Then, the precipitated solid was filtered. The substance collected by filtration was further washed with toluene-n-heptane (1:1, 8.00 mL), and then dried at 50°C under reduced pressure to obtain the title compound (1.64 g, yield: 65%).

| | |
|---|---|
| 5-Hydroxymethyl-6-(2-hydr oxy-4-benzyloxymethoxyphe nyl)-2,2,4-trimethyl-1,2-dihydroquinoline (compound 9) | ¹H-NMR (500MHz, DMSO-D₆) δ: 1.13 (s, 3H), 1.20 (s, 3H), 2.24 (d, J=1.2 Hz, 3H), 4.23-4.50 (m, 3H), 4.70 (s, 2H), 5.29 (s, 2H), 5.32-5.34 (m, 1H), 5.76 (d, J=1.2 Hz, 1H), 6.53 (d, J=8.2 Hz, 1H), 6.54 (dd, J=8.2, 2.4 Hz, 1H), 6.61 (d, J=2.4 Hz, 1H), 6.65 (d, J=8.2 Hz, 1H), 6.98 (d, J=8.2 Hz, 1H), 7.28-7.38 (m, 5H), 9.25 (brs, 1H). |
| 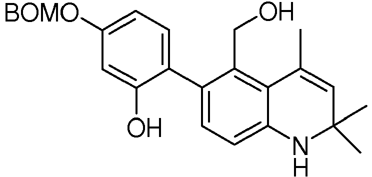 | |

### <5-Hydroxymethyl-6-(2-methoxy-4-benzyloxymethoxyphenyl)-2,2 ,4-trimethyl-1,2-dihydroquinoline (compound 10)>

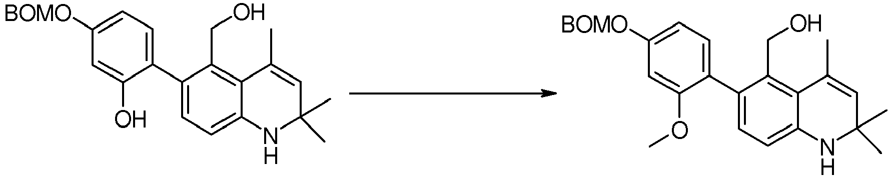

A solution obtained by mixing potassium carbonate (0.96 g, 6.95 mmol) and water (1.14 mL) with a solution of 5-hydroxymethyl-6-(2-hydroxy-4-benzyloxymethoxyphenyl)-2,2, 4-trimethyl-1,2-dihydroquinoline (1.50 g, 3.48 mmol) in N,N-dimethylformamide (22.5 mL) was cooled at an external temperature of 0°C, and methyl iodide (0.23 mL, 3.69 mmol) was added thereto, followed by stirring for 22 hours and 5 minutes. To the reaction mixture, water (30.0 mL) and ethyl acetate (36.0 mL) were added, followed by phase separation. Extraction from the aqueous layer with ethyl acetate (10.0 mL) was conducted twice. All the organic layers were combined, and washed with water (15.0 mL) three times. The organic layer was concentrated, and then an operation in which toluene (4.50 mL) was added and the mixture was concentrated was conducted three times. Then, toluene (2.50 mL) and n-heptane (5.00 mL) were added thereto followed by stirring at an external temperature of 4°C for 20 hours and 20 minutes. Then, the precipitated solid was filtered. The substance collected by filtration was further washed with a toluene-n-heptane (1:4, 10.0 mL) mixture solution, and then dried to obtain the title compound (1.26 g, yield: 81%).

| | |
|---|---|
| 5-Hydroxymethyl-6-(2-meth oxy-4-benzyloxymethoxyphe nyl)-2,2,4-trimethyl-1,2-dihydroquinoline (compound 10) | ¹H-NMR (400MHz, DMSO-D₆) δ: 1.13 (s, 3H), 1.20 (s, 3H), 2.23 (d, J=1.2 Hz, 3H), 3.65 (s, 3H), 4.15 (dd, J=12.2, 4.9 Hz, 1H), 4.32 (dd, J=4.9, 4.9 Hz, 1H), 4.46 (dd, J=12.2, 4.9 Hz, 1H), 4.73 (s, 2H), 5.31-5.33 (m, 1H), 5.36 (s, 2H), 5.78 (d, J=1.2 Hz, 1H), 6.51 (d, J=8.3 Hz, 1H), 6.63 (d, J=8.3 Hz, 1H), 6.66-6.71 (m, 2H), 7.06 (d, J=8.3 Hz, 1H), 7.26-7.40 (m, 5H). |
| 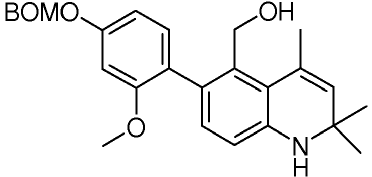 | |

### [Comparative Example]

Comparative Example in which the hydroxy group on the phenyl group in the 6-position of the 1,2-dihydroquinoline derivative was protected with a benzoyl group is shown below.

### <8-Benzoyloxy-2,2,4-trimethyl-1,2-dihydro-6-oxa-1-azachryse n-5-one (comparative compound 1)>

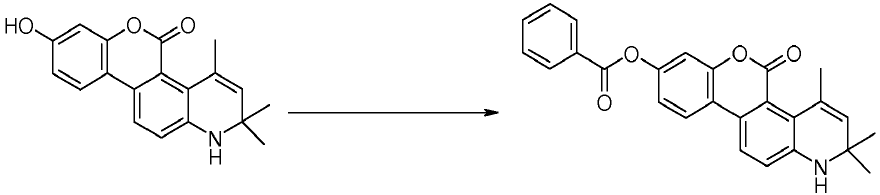

A mixture of 8-hydroxy-2,2,4-trimethyl-1,2-dihydro-6-oxa-1-azachrysen-5-one (Patent Literature 1, 2.00 g, 6.51 mmol), tetrahydrofuran (20.0 mL), and triethylamine (2.00 mL, 14.4 mmol) was stirred under ice-cooling. When the internal temperature reached 5°C, benzoyl chloride (0.82 mL, 7.12 mmol) was added, followed by stirring for 40 minutes. The temperature was returned to room temperature, followed by stirring for 2 hours and 7 minutes. The reaction mixture was ice cooled. When the internal temperature was at 6°C, water was added until a solid precipitated. The solid in the reaction mixture was filtered, washed with water (30.0 mL), and dried at 50°C under reduced pressure to obtain the title compound (2.14 g, yield: 80%).

| | |
|---|---|
| 8-Benzoyloxy-2,2,4-trimethyl-1, 2-dihydro-6-oxa-1-azachrysen-5-one (comparative compound 1) | ¹H-NMR (400MHz, DMSO-D₆) δ: 1.24 (s, 6H), 1.97 (d, J=1.0 Hz, 3H), 4.72 (s, 2H), 5.46-5.48 (m, 1H), 6.99 (s, 1H), 7.20 (d, J=8.5 Hz, 1H), 7.27 (dd, J=8.5, 2.2 Hz, 1H), 7.37 (d, J=2.2 Hz, 1H), 7.64 (dd, J=7.8, 7.8 Hz, 2H), 7.78 (tt, J=7.8, 1.5 Hz, 1H), 8.02 (d, J=9.0 Hz, 1H), 8.17 (dd, J=7.8, 1.5 Hz, 2H), 8.21 (dd, J=9.0 Hz, 1H). |
| 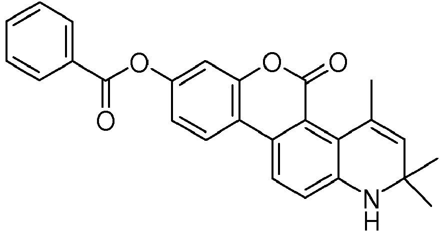 | |

### <8-Hydroxy-2,2,4-trimethyl-1,2-dihydro-6-oxa-1-azachrysen-5 -one>

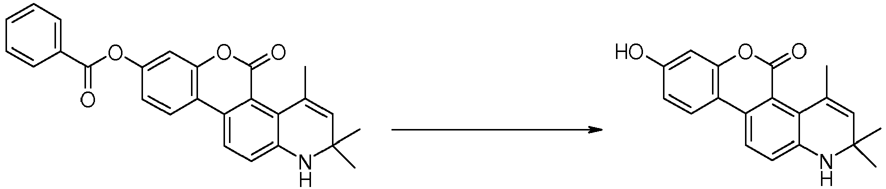

A mixture of 8-benzoyloxy-2,2,4-trimethyl-1,2-dihydro-6-oxa-1-azachrysen-5-one (1.00 g, 2.43 mmol) and anhydrous tetrahydrofuran (15.0 mL) was stirred under ice-cooling. When the internal temperature reached 0.9°C, sodium bis(2-methoxyethoxy) aluminum hydride-70% toluene solution (2.12 g, 7.34 mmol) was added thereto, followed by stirring for 1 hour and 42 minutes. Extraction from the reaction mixture was conducted by adding a mixture solution of potassium sodium tartrate tetrahydrate (2.20 g, 7.80 mmol) and water (20.0 mL) and ethyl acetate (20 ml) . Extraction from the aqueous layer with ethyl acetate (20 ml) was conducted four times. Then, the organic layers were combined, and washed with water (25 ml) twice. The organic layer was concentrated, and the residue obtained by concentration was purified by silica gel column chromatography (ethyl acetate-hexane) to obtain the title compound (0.67 g, yield: 90%).

In sum, the reduction treatment of the compound (comparative compound 1) in which the hydroxy group on the phenyl group in the 6-position of the 1,2-dihydroquinoline derivative was protected with a benzoyl group gave the compound in which the benzoyl group was removed.

| | |
|---|---|
| 8-Hydroxy-2,2,4-trimeth yl-1,2-dihydro-6-oxa-1-azachrysen-5-one | ¹H-NMR (400MHz, DMSO-D₆) δ: 1.21 (s, 6H), 1.94 (s, 3H), 5.41 (s, 1H), 6.66 (s, 1H), 6.73 (s, 1H), 6.75 (d, J=8.6 Hz, 1H), 7.13 (d, J=8.6 Hz, 1H), 7.85 (d, J=8.6 Hz, 1H), 7.91 (d, J=8.6 Hz, 1H), 9.98 (s, 1H). |
| 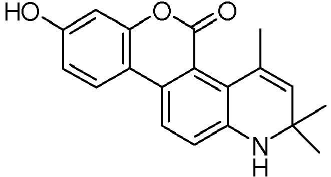 | |

### Industrial Applicability

A according to the present invention, the hydroxy group on the phenyl group in the 6-position of a 1, 2-dihydroquinoline derivative or a salt thereof is protected with a specific protective group such as a p-methoxybenzyl group, a benzyl group, or a benzyloxymethyl group. This is useful, because the need for a column chromatography purification step of each production intermediate is eliminated, and hence a compound represented by formula (7) having binding activity to the glucocorticoid receptor or a salt thereof can be industrially produced at a good yield by isolating and purifying each production intermediate by filtering and washing steps. In addition, the use of the protective group is useful, because the hydroxy group on the 6-phenyl group can be protected and deprotected at high yields.

In addition, the compound represented by formula (7) or a salt thereof is useful as an active ingredient of a prophylactic or therapeutic agent for inflammatory diseases and immune diseases, as described in Patent Literature 2.

## Claims

1. A method for producing a compound represented by formula (7) or a salt thereof: 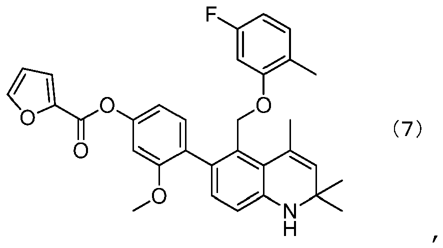 the method comprising the steps of:
removing a R¹ group and a R² group from a compound represented by formula (5) or a salt thereof, provided that the R¹ group is removed only if the R¹ group is not a hydrogen atom: 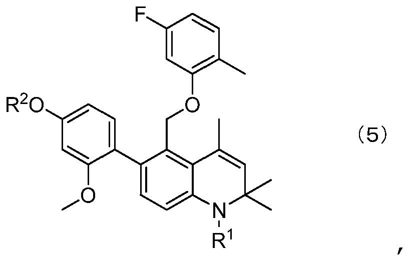 wherein R¹ represents a hydrogen atom or the same group as R², and R² represents an arylalkyl group, an arylalkyloxyalkyl group, or a substituted silyl group; and
reacting a compound represented by formula (6) or a salt thereof obtained in the removal step: 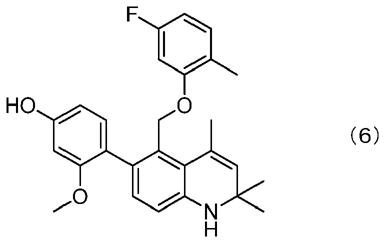 with a compound represented by formula (d) or a salt thereof: 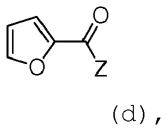 wherein Z represents a leaving group, in the presence of a base.

2. The production method according to claim 1, wherein
the compound represented by formula (5) or the salt thereof is produced by reacting a compound represented by formula (4) or a salt thereof: 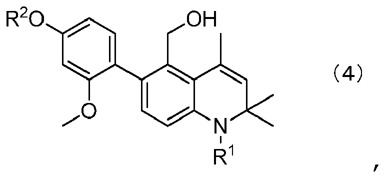 wherein R¹ and R² are as defined in formula (5),
with a compound represented by formula (c) or a salt thereof: 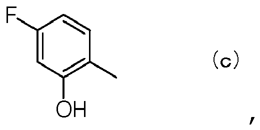 in the presence of a phosphine compound and a diazo compound.

3. The production method according to claim 2, wherein
the compound represented by formula (4) or the salt thereof is produced by reacting a compound represented by formula (3) or a salt thereof: 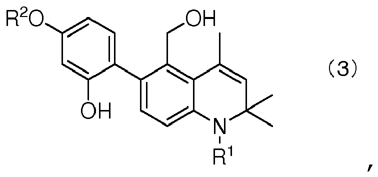 wherein R¹ and R² are as defined in formula (5),
with a compound represented by formula (b) or a salt thereof: MeY (b),
wherein Me represents a methyl group and Y represents a leaving group,
in the presence of an acid or a base.

4. The production method according to claim 3, wherein
the compound represented by formula (3) or the salt thereof is produced by subjecting a compound represented by formula (2) or a salt thereof: 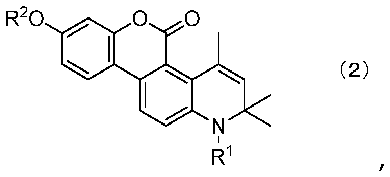 wherein R¹ and R² are as defined in formula (5),
to a reduction treatment using a reducing agent.

5. The production method according to claim 4, wherein
the compound represented by formula (2) or the salt thereof is produced by reacting a compound represented by formula (1) or a salt thereof: 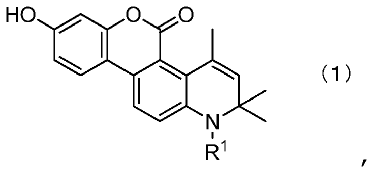 wherein R¹ represents a hydrogen atom,
with a compound represented by formula (a) or a salt thereof:
R²X (a),
wherein R² is as defined in formula (5) and X represents a leaving group,
in the presence of at least one selected from the group consisting of acids, bases, and halides.

6. The production method according to any one of claims 1 to 5, wherein
R² is a p-methoxybenzyl group, a benzyl group, or a benzyloxymethyl group.

7. The production method according to claim 1, wherein
the reaction for removing the R¹ group and the R² group is a reaction under an acidic condition or a catalytic reduction reaction in the presence of hydrogen.

8. The production method according to claim 2, wherein
the phosphine compound is triphenylphosphine or tributylphosphine, and the diazo compound is diethyl azodicarboxylate or diisopropyl azodicarboxylate.

9. The production method according to claim 4, wherein
the reducing agent is lithium aluminum hydride or sodium bis(2-methoxyethoxy)aluminum hydride.

10. A compound represented by formula (2) or a salt thereof: 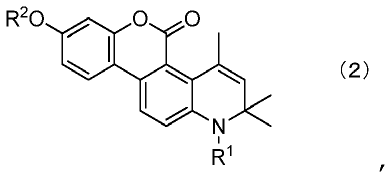 wherein R² represents an arylalkyl group, an arylalkyloxyalkyl group, or a substituted silyl group, and R¹ represents a hydrogen atom or the same group as R².

11. A compound represented by formula (3) or a salt thereof: 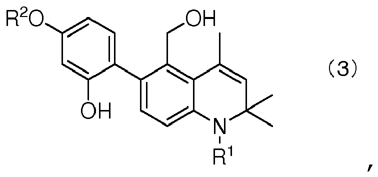 wherein R² represents an arylalkyl group, an arylalkyloxyalkyl group, or a substituted silyl group, and R¹ represents a hydrogen atom or the same group as R².

12. A compound represented by formula (4) or a salt thereof: 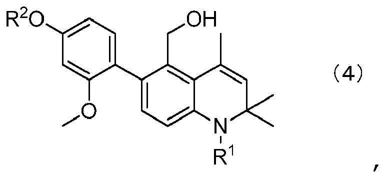 wherein R² represents an arylalkyl group, an arylalkyloxyalkyl group, or a substituted silyl group, and R¹ represents a hydrogen atom or the same group as R².

13. A compound represented by formula (5) or a salt thereof: 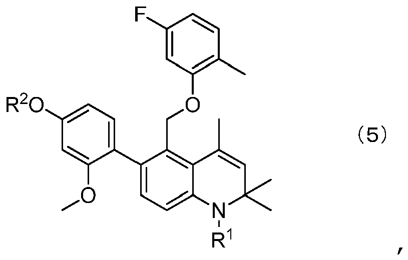 wherein R² represents an arylalkyl group, an arylalkyloxyalkyl group, or a substituted silyl group, and R¹ represents a hydrogen atom or the same group as R².

14. The compound according to any one of claims 10 to 13, or a salt thereof, wherein
R² is a p-methoxybenzyl group, a benzyl group, or a benzyloxymethyl group.

15. A compound or a salt thereof, the compound selected from the group consisting of
8-(4-methoxybenzyloxy)-2,2,4-trimethyl-1,2-dihydro-6-oxa-1-azachrysen-5-one,
6-[2-hydroxy-4-(4-methoxybenzyloxy)phenyl]-5-hydroxymethyl-1,2-dihydro-2,2,4-trimethylquinoline,
5-hydroxymethyl-6-[2-methoxy-4-(4-methoxybenzyloxy)phenyl]-1,2-dihydro-2,2,4-trimethylquinoline,
[5-[(5-fluoro-2-methylphenoxy)methyl]-6-[(2-methoxy-4-(4-me thoxybenzyloxy)phenyl)]-1,2-dihydro-2,2,4-trimethyl quinoline, and
[5-[(5-fluoro-2-methylphenoxy)methyl]-6-(4-hydroxy-2-methox yphenyl)-1,2-dihydro-2,2,4-trimethylquinoline,.
